# EUROPÄISCHE PATENTSCHRIFT

(11) **EP 2 016 962 B1**
(45) Veröffentlichungstag und Bekanntmachung des Hinweises auf die Patenterteilung: **02.07.2014**
(21) Anmeldenummer: 08160772.3
(22) Anmeldetag: 18.07.2008
(51) Int. Cl.: A61M 5/315, A61C 5/06, A61M 5/31, A61C 5/04

(54) **Spritze und Verfahren zum dosierten Abgeben von Werkstoffen**
Spray and method for metered release of material
Seringue et procédé de dépôt dosé de substances actives

(30) Priorität: 20.07.2007 DE 102007034477
(43) Veröffentlichungstag der Anmeldung: 21.01.2009
(73) Patentinhaber: VOCO GmbH, 27472 Cuxhaven (DE)
(72) Erfinder: Leiner, Uwe, 27632, Midlum (DE); Plaumann, Manfred Thomas, 27476, Cuxhaven (DE)
(74) Vertreter: Eisenführ Speiser

(56) Entgegenhaltungen:
- DE-A1- 2 025 379
- DE-A1- 4 219 502
- DE-T2- 60 212 527
- FR-A- 1 108 413

## Beschreibung

Die vorliegende Erfindung betrifft eine Spritze und ein Verfahren zum dosierten Abgeben von Werkstoffen, insbesondere (aber nicht ausschließlich) von fließfähigen und/oder pastösen dentalen Werkstoffen.

Im Dentalbereich gibt es verschiedene Füllungsmaterialien. Die Viskositäten dieser Materialien reichen von sehr festen, stopfbaren Kompositen bis hin zu sogenannten Flow-Materialien, die eine fließfähige Konsistenz aufweisen. Die fließfähigen Materialien werden üblicherweise in vorgefüllten Spritzen in Verkehr gebracht. Eine solche Spritze enthält Material für eine Vielzahl von Anwendungen. Normalerweise werden zudem Einwegkanülen zugegeben, die eine gezielte Applikation auf einen zu behandelnden Zahn ermöglichen.

Auch in anderen Bereichen werden fließfähige und/oder pastöse Werkstoffe aus einer Spritze oder ähnlichem abgegeben. Beispiele hierzu finden sich beim Löten, beim Aufbringen von Pasten wie beispielsweise einer Wärmeleitpaste oder ganz allgemein beim Auftragen beziehungsweise Applizieren von Klebstoffen. Weitere Beispiele sind kommerzielle 2-Komponentensysteme, etwa Silkonabdichtsysteme mit sogenannten Eurokartuschen, Mixpac-Spritzen und - Kartuschen und sonstige Klebesysteme.

Spritzen werden beispielsweise in den Dokumenten DE 26 44 930, DE 39 30 817, DE 42 00 044, DE 43 32 307, DE 43 32 310, DE 89 04 429, DE 699 22 027 T2, EP 0 472 023, EP 0 645 122, JP 2000-344282, JP 02-077247, US 3,853,125, US 4,863,072, US 5,697,918 und US 2005/0222539 beschrieben.

Nachteilig bei den bekannten Spritzen ist, dass das Material nach Entlastung des Stößels oder Kolbens dazu neigt, nachzulaufen. Dies bedeutet, dass fließfähiges Material auch nach Entlastung des Kolbens aus einer zum Applizieren vorgesehenen Kanüle austritt. Hierbei bildet sich ein kleiner Tropfen am Kanülenende, der je nach Größe heruntertropfen und den Arbeitsbereich verunreinigen kann. Dies ist insbesondere im dentalen Bereich für einen behandelnden Zahnarzt ein starkes Ärgernis, da die derartig verunreinigte Stelle des Behandlungstisches mit geeigneten, in der Regel starken Lösungsmitteln gereinigt werden muss. Zudem muss die Kanüle vor einer weiteren Anwendung bei demselben Patienten abgewischt werden, was einen zusätzlichen Aufwand bedeutet. Es kann hinzukommen, dass aus der Spritze abgegebenes Material sich nach einem Austreten aus der Kanüle nicht vollständig von der Kanüle löst. Dabei ergibt sich auch ohne ein Austreten von weiterem Material ein Tropfen im Bereich des Kanülenendes. Schon dieser Tropfen als Rest von gewollt aus der Spritze ausgebrachtem Material, das noch an der Kanüle verblieben ist, kann zu einer unerwünschten Verunreinigung führen.

Es ist eine Spritze des japanischen Herstellers Kuraray bekannt, bei der ein O-Ring zur Abdichtung verwendet wird. Der Spritzenstößel weist einige kleinflächige Verdickungen in der den O-Ring vorantreibenden Fläche auf, die wohl bei einer Kraftbeaufschlagung auf dem Stößel elastisch in den O-Ring eindringen sollen. Bei Entlasten der Vorrichtung soll der O-Ring vermutlich zurückfedern und damit ein teilweises Zurückziehen des Spritzenstößels aus der Hülse der Spritze verursachen. Damit würde ein Rücksaugeffekt bewirkt werden, was ein Einsaugen von Werkstoffmaterial zurück in die Spritze darstellen würde. Die Funktionalität dieser Anordnung ist jedoch stark eingeschränkt. Zum einen ergibt sich allein aus der Ausgestaltung der Anordnung und der begrenzten Verformbarkeit des O-Rings auf diese Weise schon eine Begrenzung des Rücksaugeffektes auf nur einen kleinen Wert. Hinzu kommt, dass der verformte O-Ring zwei Möglichkeiten zur Rückverformung aufweist. Lediglich bei der einen, bei der die zwischen den Verdickungen eingedrungenen Teile des O-Rings ortfest bleiben und mit dem Heranziehen des restlichen Teils des O-Rings den Spritzenstößel zurücktreiben, wird überhaupt ein Rücksaugeffekt erreicht. Die andere Möglichkeit besteht darin, dass die Teile zwischen den Verdickungen der vorantreibenden Fläche des O-Rings zu den nichteingedrungenen Teilen zurückgezogen werden, womit bestenfalls kein Rücksaugeffekt, schlimmstenfalls allerdings,sogar ein weiter den Spritzenstößel in die Spritze eintreibender Effekt aufiritt. Der O-Ring liegt an der Außenseite des Spritzenstößels und der Innenseite der Spritzenhülse jeweils ganzflächig an, wobei die Art der Rückverformung durch die lokalen Reibungsverhältnisse bestimmt ist, insofern also im Wesentlichen dem Zufall überlassen bleibt

In EP 0 645 122 wird eine Anordnung mit einer im Ruhezustand gegenüber einem Spritzeninnenraum in Längsachsenrichtung nach außen ausgebauchten Membran zur Verwendung für hochviskose Materialien in Drehspritzen vorgeschlagen. Gemäß EP 0 645122 verformt der beim Applizieren vorangetriebene Drehkolben die Membran, sodass sie in Richtung des Spritzeninnenraums "umschlägt". Nach dem Applizieren des Materials wird der Drehkolben zurückgezogen, sodass eine Entlastung der Membran erfolgt, die eine Rückverformung der Membran in den ursprünglichen Zustand erlaubt. Hierbei ist Insbesondere nachteilig, dass es bei einem Applizieren von Material unbestimmt ist, ob die Membran als Ganzes ohne eine Verformung der Membran verschoben wird, oder ob eine Verformung der Membran zum Applizieren von Material führt. Ferner ist ein manuelles Zurückdrehen des Drehkolbens durch den Benutzer notwendig, was auch ohne eine gesonderte Membran zu einer Entlastung des Materials im Spritzeninneren führt.

DE 602 12 527 T2 betrifft eine injektoranordnung zum Verhindern von anschließendem Heraustropfen mit einem rohrförmigen Zylinder mit einem distalen Ende, an welchem ein Applikator anbringbar ist; einem Kolben, welcher in den Zylinder axial bewegbar derart eingesetzt ist, dass ein distales Ende innerhalb des Zylinders angeordnet ist und ein proximale Ende sich nach außen durch eine Öffnung erstreckt, die an dem proximalen Ende des Zylinders angeordnet ist; und einem Dichtungskörper, der an dem distalen Ende des Kolbens angeordnet ist; wobei der Dichtungskörper einen Basisabschnitt, der sich von dem Kolben aus zu dem distalen Ende des Zylinders erstreckt, und einen Flanschabschnitt aufweist, der sich von dem Basisabschnitt aus radial nach außen erstreckt und mit einem Umfangsrand versehen ist, der in allen Ebenen, die die Längsachse des Kolbens aufweisen, abgerundet ist und der einen Innenumfang des Zylinders zum Definieren eines Aufnahmeraumes innerhalb des Zylinder berührt; wobei der Flanschabschnitt mittels einer Reaktionskraft eines Materials, das innerhalb des Aufnahmeraumes untergebracht ist, elastisch verformbar ist, wenn der Dichtungskörper mittels einer auf den Kolben ausgeübten Druckkraft so in Richtung des distalen Endes des Zylinders geschoben wird, dass das Material aus dem Aufnahmeraum herausgedrückt wird und den Kolben mittels einer eigenen Rückstellkraft des Flanschabschnitts zurück in Richtung des proximalen Endes des Zylinders dann drückt, wenn die Druckkraft von dem Kolben weggenommen wird; wobei sich der Flanschabschnitt von dem Basisabschnitt aus radial nach außen erstreckt und zwischen einem Anfangszustand mit einer konkaven Fläche, die dem distalen Ende des Zylinders zugewandt ist, und einem elastisch verformten Zustand mit einer konkaven Fläche, die dem proximalen Ende des Zylinders zugewandt ist, verformbar ist.

Weitere Spritzen sind bekannt aus US 1,663,627, US 1,948,982, US 2,419,401, US 2,526,365, US 2,575,425, US 2,902,034, US 3,045,674, US 3,618,603. US 3,678,930, US 3,766,918, US 3,890,956, US 4,363,329, US 4,381,779, US 4,543,093, US 4,678,107 US 4,986,820 und US 6,796,217.

US 3,766,918 offenbart eine selbstansaugende hypodermische Spritze, bei der ein Kolben durch elastische Verformung dazu genutzt wird, ein Rückstrom oder Ansaugstrom durch die Nadel in die Hülse zu bewirken, wenn eine Kraft auf den Kolben freigegeben wird.

Aufgabe der vorliegenden Erfindung ist es, eine Spritze sowie ein Verfahren zum dosierten Abgeben von Werkstoffen bereitzustellen, bei denen der oben beschriebene Nachlaufeffekt, insbesondere bei mittel- und niederviskosen Materialien, nicht oder nur verringert auftritt und bei denen ein gewünschter, vorbestimmbarer und ausreichend großer Rücksaugeffekt in kontrollierter und reproduzierbarer Weise auftritt.

Die Aufgabe wird gelöst durch eine Spritze zum dosierten Abgeben von Werkstoffen, insbesondere von fließfähigen und/oder pastösen dentalen Werkstoffen, wie sie in Anspruch 1 definiert ist.

Die Aufgabe wird zudem gelöst durch ein Verfahren zum dosierten Abgeben von Werkstoffen wie es in Anspruch 14 definiert ist.

Die relative Verschiebung zwischen Reibschlusselement und Kolbenkörper steht in Beziehung mit der Kraft, die sich durch die Auslenkung beziehungsweise Spannung des Federelementes ergibt. Es ist von Vorteil, wenn insbesondere zur Materialschonung des Federelementes hierbei nur eine vorbestimmte maximale Auslenkung des Federelementes, also eine begrenzte Relativverschiebung zwischen Reibschlusselement und Kolbenkörper vorgesehen ist. Zudem kann durch die Begrenzung der daraus resultierenden am Federelement auftretenden Spannkraft sichergestellt werden, dass die Spannkraft nicht die am Reibschlusselement angreifende Reibkraft übersteigt und das Reibschlusselement somit ungewollt und gegebenenfalls unkontrolliert freigesetzt wird.

Bei dem erfindungsgemäßen Verfahren umfasst das Bereitstellen der Spritze bevorzugt die Schritte: Einbringen des Kolbens in die zumindest teilweise leere Spritze, Bereitstellen von Werkstoff und Ausbringen des Kolbens zur Füllung der Spritze mit Werkstoff. Das Ausbringen kann durch ein Zurückziehen des Kolben(körper)s und/oder durch ein Austreiben des Kolbens aus der Hülse erfolgen. Das Austreiben des Kolbens kann beispielsweise durch das erzwungene Befüllen der Spritze durch die Auslassöffnung hindurch über den Werkstoff bewirkt werden.

Bei der erfindungsgemäßen Spritze sind Hülse, Kolbenkörper, Reibschlusselement und Federelement so angeordnet, dass bei einem Einschieben des Kolbens und/oder des Kolbenkörpers in die Hülse (beispielsweise aufgrund einer manuellen Kraftbeaufschlagung beziehungsweise Krafteinwirkung auf den Kolbenkörper) das Reibschlusselement infolge der zwischen dem Reibschlusselement und der Innenwandung der Hülse wirkenden Reibungskraft relativ gegenüber dem Kolbenkörper zurückbleibt, wobei das Federelement hierdurch gespannt wird. Die Reibungskraft verhindert somit zumindest zeitweilig, dass sich das Reibschlusselement bei Einschieben des Kolbens bzw. Kolbenkörpers gemeinsam mit dem Kolbenkörper bewegt. Somit wird an dem Reibschlusselement im Vergleich zum Kolbenkörper eine geringere Arbeit verrichtet, wobei ein Teil der beim Einschieben verrichteten Arbeit in dem gespannten Federelement gespeichert wird.

Das Federelement koppelt das Reibschlusselement und den Kolbenkörper miteinander, sodass zumindest eine auf den Kolbenkörper zum Eindringen in die Hülse ausgeübte Kraft wenigstens teilweise über das Federelement auf das Reibschlusselement übertragen wird und beim Entlasten des Kolbenkörpers, also der Entspannung des Federelements, das Reibschlusselement als Widerlager für das Federelement wirkt, sodass das Federelement den Kolbenkörper beim Entspannen zumindest teilweise austreibt.

Für eine Kopplung ist es nicht notwendig, dass das Federelement das Reibschlusselement und den Kolbenkörper derart miteinander verbindet, dass etwa auch bei einem Zurückziehen des Kolbenkörpers das Reibschlusselement über das Federelement mitgenommen wird. Erfindungsgemäß ist eine derartige feste Kopplung allerdings nicht ausgeschlossen.

Das Federelement ist so ausgestaltet, dass es zumindest beim Betätigen des Kolbens, also dem Eindringen des Kolbenkörpers in die Hülse, wenigstens einen Teil der eingebrachten Energie als Verformungsenergie aufnehmen und in Form einer Rückstellung wieder abgeben kann.

Nach Beendigung der Kraftbeaufschlagung, d. h. bei Entlastung des Kolbens bzw. Kolbenkörpers, wirken dann infolge des gespannten Federelements Rückstellkräfte, sodass der Kolbenkörper entgegen der Einschubrichtung zumindest teilweise aus der Hülse getrieben wird. Die beim Einbringen des Kolbens bzw. des Kolbenkörpers und Spannen des Federelements in dem Federelement gespeicherte Energie wird hier dazu genutzt, den Kolbenkörper zumindest teilweise wieder aus der Hülse zu entfernen, womit der Innenraum zwischen Hülse und Kolben, in dem das zu applizierende Material, d.h. der Werkstoff, aufgenommen ist, vergrößert wird. Damit ergibt sich ein Rücksaugeffekt, der auch ein (Wieder-)Einsaugen von fließfähigem Material bewirken kann, das sich außerhalb der Spritze im Bereich der Austrittsöffnung der Hülse befindet.

Die fließfähigen und/oder pastösen Werkstoffe, für die die erfindungsgemäße Spritze und das erfindungsgemäße Verfahren bevorzugt vorgesehen sind, weisen im Scherversuch bei 23°C mit Platte/Platte Geometrie bei Scherraten von 0,1 bis 10 s⁻¹insbesondere eine Viskosität im Bereich 0,5 bis 5000 Pa·s auf.

Die Größe der Haft- bzw. Gleitreibung zwischen Hülseninnenwandung und Reibschlusselement kann durch geeignete Materialpaarungen und Einstellungen von Oberflächeneigenschaften eingestellt werden. Die grundsätzlichen Möglichkeiten in diesem Zusammenhang sind dem Fachmann bekannt, so dass auf eine detaillierte Beschreibung verzichtet werden kann. Besonders gute Ergebnisse lassen sich mit Polyolefinen (z.B. Polypropylen PP oder Polyethylen PE) gepaart mit Silikonen bzw. Elastomeren erreichen. Andere Materialpaarungen sind allerdings ebenfalls möglich.

Die Reibungskräfte bewegen sich dabei typischerweise in einer Größenordnung von 0,1 N bis 10N. Höhere Reibungskräfte ermöglichen hierbei einen kräftigeren Rücksaugeffekt, was insbesondere bei höherviskosen Materialien zweckmäßig ist, damit der Rücksaugeffekt kräftig genug ist, um sich bis durch die (ggf. enge) Applikationskanüle bis zu deren Ausgang hin auszuwirken. Niedrigere Reibungskräfte erhöhen den Anwendungskomfort der Spritze.

Das Spannen des Federelements erfolgt bevorzugt durch Einwirken einer Scherkraft, einer Zugkraft oder einer Druckkraft auf das Federelement als Ganzes, wobei, je nach Ausgestaltung des Federelements im Speziellen, innerhalb des Federelements auch andere Kraftarten auftreten können. Beispielsweise treten in einem Federelement, das wie eine bekannte Spiralfeder ausgebildet ist, im Material jeweils Torsionsspannungen auf, auch wenn das Federelement als ganzes eine Zug- oder Druckspannung aufnimmt. Kombinationen von unterschiedlichen Kraftwirkungen bzw. Spannungsaufnahmen sind ebenfalls möglich. Beispielsweise kann ein Federelement beim Spannen eine Zug- und eine Scherkraft erfahren.

Geeignete Beispiele für Materialien, aus denen das Federelement hergestellt werden kann sind: Silikone, (thermoplastische-) Elastomere, Nitrilkautschuk NBR, Ethylen-Propylen-Dien-Kautschuk EPDM und dergleichen. Besonders bevorzugt werden Silikone wegen ihrer hohen Elastizität und guten chemischen Beständigkeit.

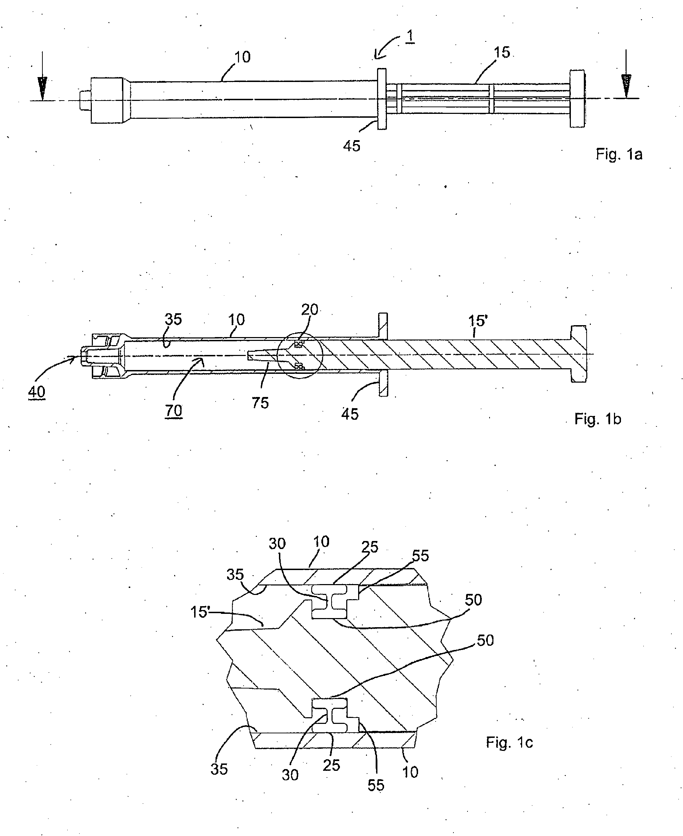

In einer Ausführungsform der Erfindung weist der Kolben ein Dichtungselement auf, das zur Abdichtung mit der Innenwandung der Hülse gegenüber einem Durchtritt von Werkstoff ausgestaltet ist (siehe beispielsweise Fig. 1c, 2c, 5c, 6c, 7c, 9c, 10c, 13c oder 15c).

Ist der Kolben mit einem Dichtungselement versehen, so kann damit, insbesondere bei dünnflüssigen Materialien, ein Austreten beziehungsweise Durchtreten von Werkstoff am Kolben vorbei aus der Spritze heraus vermieden oder zumindest ausreichend unterdrückt werden. Ein gesondertes Dichtungselement erlaubt hierbei eine bessere Abdichtungswirkung als die Dichtwirkung, die lediglich durch die geringe Breite eines Spalts zwischen Kolben und Hülse gewährleistet wird.

In einer vorteilhaften Ausführungsform der Erfindung wird das Dichtungselement von dem Reibschlusselement, dem Federelement oder einer Kombination aus Reibschlusselement und Federelement gebildet.

Reibschlusselement, Federelement oder eine Kombination aus Reibschlusselement und Federelement werden vorzugsweise eine Doppelfunktion erfüllen und zusätzlich als Dichtungselement fungieren; dies ermöglicht einen einfachen Aufbau der erfindungsgemäßen Spritze, ohne dass zusätzliche Elemente wie ein gesondertes Dichtungselement vorgesehen werden müssten.

In diesem Zusammenhang sei darauf hingewiesen, dass, auch wenn das Reibschlusselement erfindungsgemäß an einer Innenwandung beziehungsweise Innenfläche der Hülse reibschlüssig anliegt, damit nicht notwendigerweise verbunden ist, dass das Reibschlusselement über den gesamten Umfang der Innenwandung an dieser anliegt. Es kann auch vorgesehen sein, dass das Reibschlusselement lediglich abschnittsweise, also nur in bestimmten (Teil-)Bereichen, an der Innenwandung der Hülse anliegt. Bei geeigneter Wahl der Materialparameter und bei geeigneter Geometrie von Innenwandung und Reibschlusselement wird auch ohne vollflächiges Anliegen eine ausreichende Reibkraft sichergestellt.

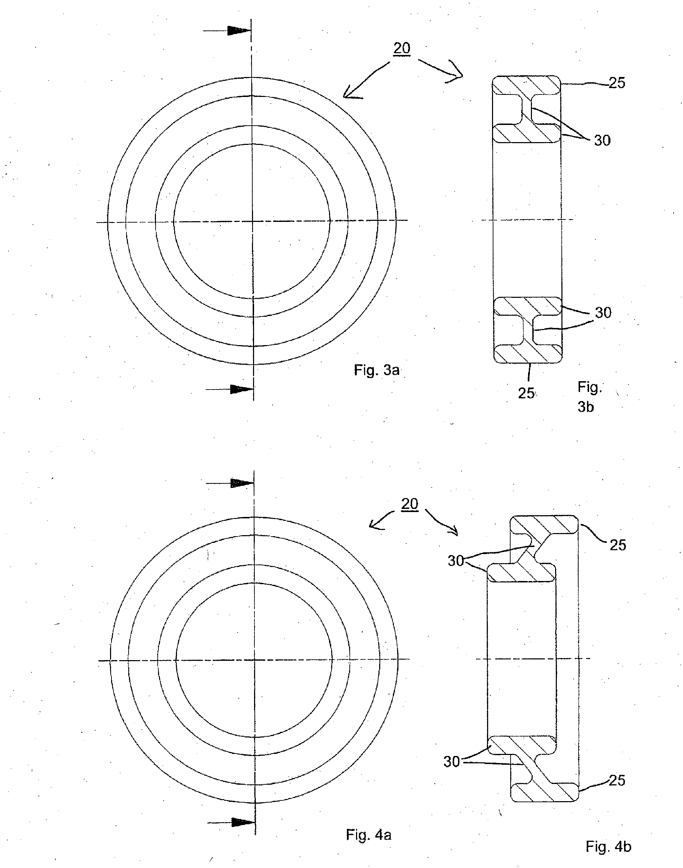

Gemäß einer vorteilhaften Ausführungsform verbindet das Federelement Reibschlusselement und Kolbenkörper miteinander (siehe beispielsweise Fig. 3b oder 4b).

Dient das Federelement nicht nur zur Erzeugung einer Federwirkung, sondern zusätzlich auch zur Verbindung von Reibschlusselement und Kolbenkörper, wird eine einfache Kraftübertragung auf den Kolbenkörper erreicht, wobei das Reibschlusselement für die Kraftwirkung gewissermaßen als Widerlager dient, ohne das zusätzliche Elemente erforderlich wären. Weitere Elemente sind aber auch nicht ausgeschlossen.

Zudem kann durch eine Verbindung von Reibschlusselement und Kolbenkörper über das Federelement miteinander eine relative Fixierung von Reibschlusselement und Kolbenkörper erreicht werden, in dem Sinne, dass beispielsweise bei einem Herausziehen des Kolbenkörpers aus der Hülse über das Federelement das Reibschlusselement mit aus der Hülse entfernt wird. Allerdings ist bei der obigen Ausführungsform nicht zwingend vorgesehen, dass eine feste Verbindung zwischen Federelement, Reibschlusselement und Kolbenkörper vorgesehen ist. Die Verbindung kann sich auch lediglich dadurch ergeben, dass das Federelement zwischen Reibschlusselement und Kolbenkörper angeordnet ist und somit als Verbindung zwischen diesen Elementen dient.

Nach einer anderen Ausführungsform der vorliegenden Erfindung ist das Federelement bei in die Hülse eingebrachten Kolben durch das Reibschlusselement von der Innenwandung der Hülse beabstandet (siehe beispielsweise Fig.1c, 2c,5c, 6c, 7c, 9c, 10c, 13c oder 15c).

Wenn sich das Reibschlusselement zwischen der Innenwandung der Hülse und dem Federelement befindet und diese voneinander trennt, ist eine unmittelbare Beeinflussung des Federelements durch die Innenwandung ausgeschlossen. Damit ist sichergestellt, dass nur bei relativer Bewegung zwischen dem Reibschlusselement und dem Kolbenkörper beim Einbringen beziehungsweise Herausziehen des Kolbens bzw. Kolbenkörpers in die Hülse eine Krafteinwirkung auf das Federelement erfolgt, nicht aber durch ein Bewegen des Kolbens, das nicht zu einer Relativbewegung von Reibschlusselement und Kolbenkörper führt.

In einer bevorzugten Ausführungsform der Erfindung sind das Reibschlusselement und das Federelement gemeinsam einstückig (als Reibschlussfederelement) ausgebildet (siehe beispielsweise Fig. 3a, 3b, 4a, 4b, oder 8a-d).

Bei einer Ausführung eines Reibschlussfederelementes als einstückigem Element, das sowohl die Funktion des Reibschlusselementes als auch die Funktion des Federelementes übernimmt, kann eine vergleichsweise vereinfachte Herstellungsweise von Reibschlusselement und Federelement eingesetzt werden, bei der auf eventuelle sonstig nötige Verbindungselemente oder Verbindungsarbeitsschritte verzichtet werden kann.

Gemäß einer vorteilhaften Ausführungsform ist das Federelement an dem Kolbenkörper verankert.

Bei einer Fixierung oder Befestigung des Federelementes am Kolbenkörper, insbesondere bei Fixierung gegenüber einer relativen Verschiebung in Richtung der Längsachse der Spritze, kann unabhängig von einer eventuellen Bewegungsrichtung, etwa beim Einbringen und dem Herausziehen des Kolbens sichergestellt werden, dass Federelement und Kolbenkörper relativ zueinander unbewegt bleiben (zumindest in Längsrichtung) und sich somit durchgehend in einer gewünschten örtlichen Beziehung zueinander befinden.

Andere mögliche Beispiele für eine Fixierung von Federelement und Kolbenkörper zueinander sind die bekannten Fügemethoden Kleben oder Schweißen.

Gemäß einer weiteren Ausführungsform sind Kolbenkörper, Reibschlusselement und Federelement gemeinsam einstückig ausgebildet.

Ein einstückiger Kolben mit integralem Kolbenkörper, Reibschlusselement und Federelement ist ohne weitere Montageschritte sofort einsetzbar. Selbst wenn die Herstellung eines einstückigen Kolbens im Vergleich zur Herstellung der Komponenten Kolbenkörper, Reibschlusselement und Federelement unter Umständen komplexer sein kann, erlaubt eine einstückige Ausführung dennoch eine einfachere Logistik, da zur Montage der Spritze der Kolben nicht noch aus Einzelteilen zusammengesetzt oder kombiniert werden müsste.

Bei einer bevorzugten Ausführung ist der Kolben mit Kolbenkörper, Reibschlusselement und Federelement mit einem 2- oder Mehr-Komponenten-Spritzgussverfahren hergestellt.

Das an sich bekannte 2- bzw. Mehrkomponenten-Spritzgussverfahren erlaubt eine Herstellung einstückiger Bauteile, bei denen jeweils Teile oder Abschnitte, z.B. infolge unterschiedlicher Einsatzmaterialien, unterschiedliche Eigenschaften aufweisen. So können auch bei einer einstückigen Ausführung des Kolbens jeweils beispielsweise eine gewünschte Elastizität des Federelements, eine gewünschte Festigkeit des Kolbenkörpers und/oder gewünschte Reibungseigenschaften des Reibschlusselements weitgehend unabhängig voneinander eingestellt werden. Bei geeignetem Einsatz moderner Fertigungstechniken wie beispielsweise dem 2-Komponenten-Spritzguß ist möglich, dass der Bereich mit dem Federelement aus einem vergleichsweise flexiblen Werkstoff und der angrenzende, den Kolbenkörper bildende Bereich aus einem vergleichsweise formbeständigen Werkstoff bestehen kann.

Eine weitere erfindungsgemäße Alternative zu dem obigen besteht darin, dass (lediglich) das Federelement und der Kolbenkörper gemeinsam einstückig ausgebildet sind. Hierbei ist das Reibschlusselement gesondert ausgebildet und wird mit Federelement (und Kolbenkörper) bei einem Zusammenbau der erfindungsgemäßen Spritze zusammengebracht. Wie auch im Fall des einstückigen Kolbens und auch beim einstückigen Reibschlussfederelement ist das bevorzugte Herstellungsverfahren ein 2- bzw. Mehrkomponenten-Spritzgussverfahren.

In einer möglichen Ausführungsform der Erfindung erstreckt sich das Federelement in einer Ebene senkrecht zu einer Längsachse des Kolbenkörpers um den Kolbenkörper und liegt an einer Außenfläche des Kolbenkörpers an, wobei das Federelement insbesondere an der Außenfläche verankert ist (siehe beispielsweise Fig. 1c, 2c, 2d, 9c, 13c oder 15c).

Eine Möglichkeit der Verankerung des Federelementes an dem Kolbenkörper besteht darin, das Federelement auf der Außenfläche des Kolbenkörpers anliegen zu lassen, wobei sich das Federelement im Wesentlichen senkrecht zu einer Längsachse des Kolbenkörpers erstreckt. Hierbei kann sich insbesondere, wie oben erläutert, zwischen dem Federelement und der Innenwandung der Hülse das Reibschlusselement befinden, wobei infolge einer Relativbewegung zwischen Reibschlusselement und Kolbenkörper eine quer zu einer Ebene des Federelementes wirkende Kraft auftritt, mit der das Federelement gespannt wird.

In einer vorteilhaften Ausführungsform der Erfindung ist das Federelement zumindest teilweise in einer Ausnehmung des Kolbenkörpers aufgenommen (siehe beispielsweise Fig. 1c, 2c, 2d, 9c, 13c oder 15c).

Wenn das Federelement zumindest teilweise in einer Ausnehmung des Kolbenkörpers aufgenommen ist, kann durch diese räumliche Anordnung auf einfache Weise ein Verschieben des Federelementes entlang einer Längsachse des Kolbenkörpers verhindert werden, da die Ausnehmung mittels ihrer Begrenzung eine solche Verschiebung verhindert. Der Kolbenkörper weist also vorzugsweise eine Ausnehmung zur zumindest teilweisen Aufnahme des Federelements auf, wobei wenigstens eine Ausnehmungsbegrenzung eine Längsverschiebung des Federelements verhindert, so dass das Federelement in Längsrichtung in der Ausnehmung fixiert ist.

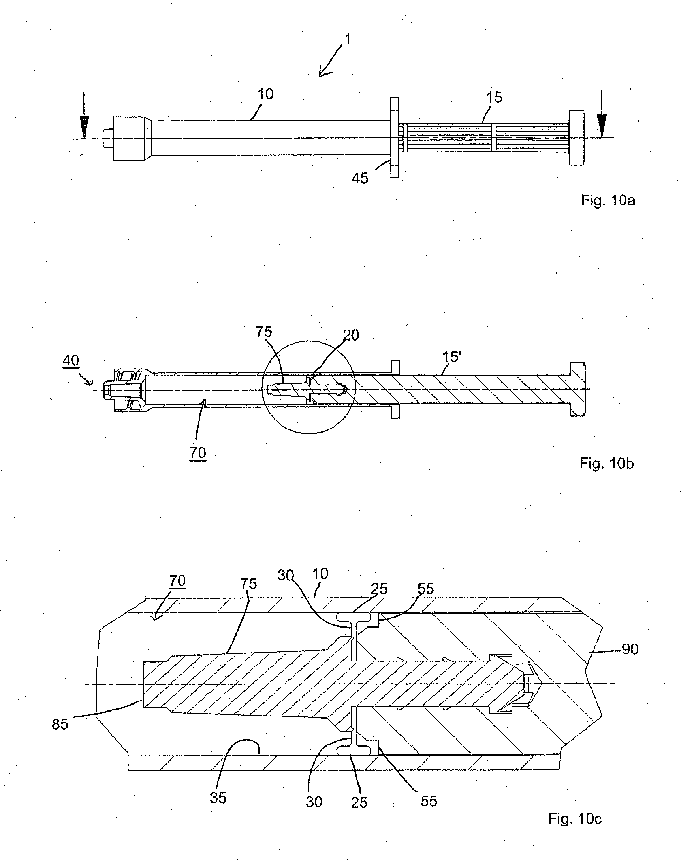

In einer weiteren Ausführungsform ist das Federelement im Kolbenkörper, insbesondere zwischen einem ersten und einem zweiten Teil des Kolbenkörpers, eingespannt (siehe beispielsweise Fig. 10c).

Eine weitere Möglichkeit der Verankerung des Federelementes am Kolbenkörper besteht in einer form- und/oder kraftschlüssigen Fixierung durch ein Einspannen des Federelementes im Kolbenkörper. Bei einem geeigneten Einspannen des Federelements im Kolbenkörper erfolgt eine einfachere Montage des erfindungsgemäßen Kolbens bzw. der erfindungsgemäßen Spritze im Vergleich zu einer Montage, bei der das Federelement auf einen Kolbenkörper aufgezogen und in eine Ausnehmung des Kolbenkörpers eingeführt werden muss. Durch die Ausgestaltung von Federelement und Kolbenkörper wird ein ungewolltes Lösen, insbesondere ein Herausziehen, des Federelements aus dem Kolbenkörper verhindert.

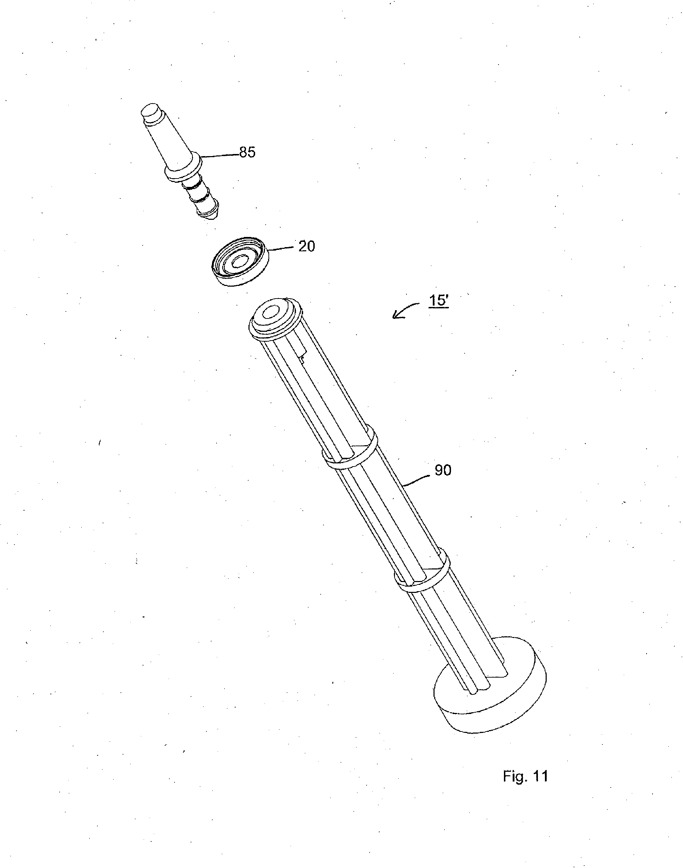

Gemäß einer bevorzugten Ausführungsform weist das Federelement eine Öffnung, insbesondere eine mittige Öffnung, auf, durch die sich der erste Teil des Kolbenkörpers erstreckt, wobei der erste Teil des Kolbenkörpers und der zweite Teil des Kolbenkörpers miteinander verbunden sind (siehe beispielsweise Fig. 10c oder 11).

Insbesondere wenn die Einspannung des Federelementes zwischen einem ersten und einem zweiten Teil des Kolbenkörpers durch eine Öffnung des Federelementes erfolgt, kann durch eine geeignete Verbindung des ersten und zweiten Teils des Kolbenkörpers ein Einspannen des Federelementes erreicht werden. Werden das erste und zweite Teil des Kolbenkörpers hierbei so miteinander verbunden, dass eine geeignete Fixierungskraft auf das Federelement ausgeübt wird, ergibt sich somit eine kraftschlüssige Verbindung von Federelement und Kolbenkörper. Eine Alternative oder Ergänzung kann darin bestehen, dass das Federelement durch eine geeignete räumliche Ausgestaltung von Federelement und ersten beziehungsweise zweiten Teil des Kolbenkörpers oder des Kolbenkörpers allgemein durch eine formschlüssige Verbindung verankert wird.

Bei der obigen bevorzugten Ausführungsform können in vorteilhafter Weise der erste Teil und der zweite Teil des Kolbenkörpers mittels einer Schnapp- und/oder einer Schraubverbindung miteinander verbunden sein.

Durch eine Schnapp- und/oder Schraubverbindung von erstem und zweiten Teil des Kolbenkörpers kann eine einfache Montage beziehungsweise Verankerung des Federelementes am Kolbenkörper erreicht werden. Hierbei ist es insbesondere nicht nötig, allerdings möglich, dass die Verbindung zwischen ersten und zweiten Teil des Kolbenkörpers zerstörungsfrei lösbar ist. Auch andere Möglichkeiten der Verbindung, zu denen beispielsweise eine Schweiß- und/oder Klebverbindung gehört, sind vorstellbar.

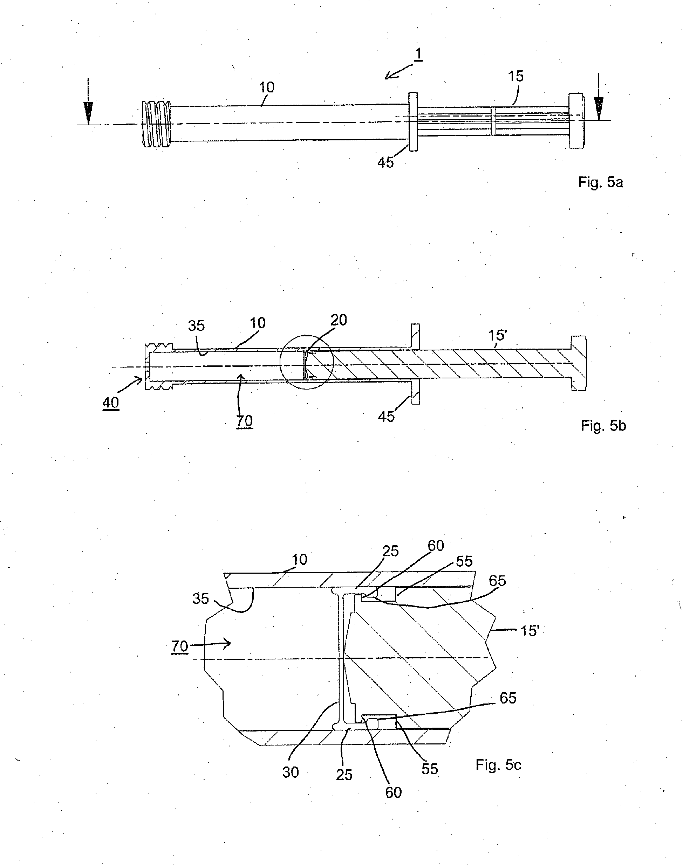

In einer bevorzugten Ausführungsform der Erfindung sind das Federelement und das Reibschlusselement bei in die Hülse eingebrachtem Kolben zwischen dem Hülseninnenraum und dem Kolbenkörper angeordnet (siehe beispielsweise Fig. 5c, 6c oder 7c).

Alternativ oder ergänzend zu einer Verankerung des Federelementes am Kolbenkörper kann das Federelement (vorzugsweise auch zusammen mit dem Reibschlusselement) in Einbringrichtung vor dem Kolbenkörper vorgesehen sein, sodass ein Einbringen des Kolbenkörpers in die Hülse eine entsprechend versetzte Verschiebung des Reibschlusselementes beziehungsweise eine gewünschte Spannung des Federelementes zur Folge hat.

Gemäß einer erfindungsgemäßen Ausführungsform weist die Hülse eine sich verjüngende Auslassöffnung auf, wobei der Kolben mit einer an die Auslassöffnung angepassten Spitze versehen ist.

Ein Ausbringen von in der Hülse aufgenommenem Werkstoff kann in vielen Fällen durch eine sich verjüngende beziehungsweise gegenüber dem Hülsenquerschnitt verkleinerte Auslassöffnung verbessert werden, wobei es sich insbesondere für eine Befüllung der Spritze von vorn, d. h. durch die Auslassöffnung, als vorteilhaft zeigt, wenn der Kolben mit einer geeigneten Spitze versehen ist, die den Innenbereich der Auslassöffnung ausfüllt.

In einer bevorzugten Ausführungsform der Erfindung weist die Spritze, insbesondere der Kolbenkörper ein erstes Begrenzungselement zur Begrenzung der Relativverschiebung zwischen Reibschlusselement und Kolbenkörper beim Einbringen des Kolbens in die Hülse auf, insbesondere eine Stufe oder Kante, an die das Reibschlusselement und/oder das Federelement bei gespanntem Federelement anlegbar sind.

Bei einem Einschieben oder Einbringen des Kolbens und/oder des Kolbenkörpers in die Hülse wird vorzugsweise durch Vorsehen beispielsweise einer Stufe oder Kante am Kolbenkörper gegebenenfalls ein Anlegen beziehungsweise Anstoßen von Reibschlusselement und/oder Federelement erreicht, was auf einfache Weise die Relativverschiebung zwischen Kolbenkörper und Reibschlusselement begrenzt.

In einer weiteren bevorzugten Ausführungsform der Erfindung weist die Spritze, insbesondere der Kolbenkörper, ein zweites Begrenzungselement zur Begrenzung der Relativverschiebung bei einem zumindest teilweisen Entfernen des Kolbens und/oder des Kolbenkörpers aus der Hülse auf, wobei der Kolbenkörper insbesondere mit einem ersten Vorsprung versehen ist, der einen zweiten Vorsprung des Reibschlusselements und/oder des Federelements hintergreift, so dass der erste Vorsprung bei dem Entfernen des Kolbens an den zweiten Vorsprung anlegbar ist (siehe beispielsweise Fig. 5c, 6c, 7c).

Wenn Kolbenkörper und Reibschlusselement so ausgestaltet sind, dass ein Herausziehen des Reibschlusselementes aus der Umgebung des Kolbenkörpers bei einem Herausziehen des Kolbenkörpers beziehungsweise des Kolbens aus der Hülse vermieden wird, kann beispielsweise auch das Federelement unabhängig von einer Fixierung am Kolbenkörper vorgesehen sein, beispielsweise als scheibenförmiges Element zwischen Kolbenkörper und Hülseninnenraum. Durch das räumliche Ineinandergreifen von Reibschlusselement und Kolbenkörper ist damit auch bei einem Herausziehen des Kolbens aus der Hülse eine vorbestimmte relative räumliche Anordnung von Reibschlusselement, Federelement und Kolbenkörper zueinander gewährleistet. Ein solches Herausziehen wird beispielsweise vorgenommen, wenn die Hülse durch die Auslassöffnung hindurch manuell mit Werkstoff befüllt wird.

In einer vorteilhaften Ausführungsform der Erfindung ist das Federelement beim Einbringen des Kolbens und/oder des Kolbenkörpers durch eine Scherkraft, eine Zugkraft und/oder durch eine Presskraft spannbar.

In vorteilhafter Weise kann mit einfachen Federelementen ein Scheren, Ziehen oder Pressen des Federelementes zur Ausübung der Kraft beziehungsweise zur Energiespeicherung im Federelement genutzt werden. Andere Arten der Krafteinwirkung bzw. -speicherung sind ebenfalls möglich.

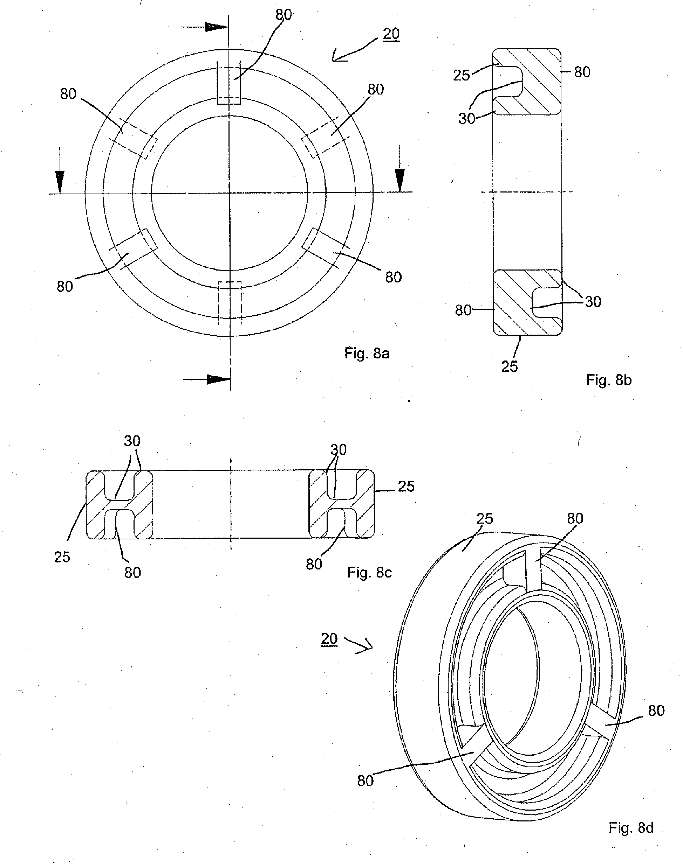

Gemäß noch einer weiteren Ausführungsform der Erfindung weist das Federelement wenigstens ein Versteifungselement auf, insbesondere mehrere Versteifungselemente, die regelmäßig über das Federelement verteilt angeordnet sind (siehe beispielsweise Fig. 8a-d).

Mit dem Vorsehen von einem oder mehreren Versteifungselementen im Federelement kann eine gewünschte Spannungscharakteristik unter größerer Unabhängigkeit von einzelnen räumlichen Ausgestaltungen des Federelementes erreicht werden.

In einer weiteren vorteilhaften Ausführungsform der vorliegenden Erfindung weist die Hülse eine Auslassöffnung mit einem Ansatz zur Aufnahme eines Füllrohres auf, wobei der Ansatz sich von außen in Richtung auf den Hülseninnenraum verjüngt, insbesondere konisch verjüngt. Besonders bevorzugt wird eine konische Verjüngung mit einem Winkel von 0,25 bis 2° gegenüber der Mittelachse, insbesondere mit einem Winkel von 0,5° (entsprechend einem Kegelwinkelbereich von 0,5° bis 4° bzw. einem Kegelwinkel von 1 °).

Die Erfindung betrifft ferner einen Kolben für eine Spritze zum dosierten Abgeben von Werkstoffen, insbesondere von fließfähigen und/oder pastösen dentalen Werkstoffen, bevorzugt für eine Spritze wie oben beschrieben oder zum Einsatz in einem Verfahren wie oben beschrieben, wobei der Kolben in die Spritze einbringbar ist und einen Kolbenkörper, ein Reibschlusselement, das an einer Innenwandung einer Hülse der Spritze reibschlüssig anlegbar ist, und ein das Reibschlusselement und den Kolbenkörper koppelndes Federelement aufweist, wobei der Kolben ausgestaltet ist, bei in die Hülse eingebrachtem Kolben mit der Hülse einen Hülseninnenraum zur Aufnahme des Werkstoffs begrenzen, wobei das Federelement bei einem Einbringen des Kolbens und/oder des Kolbenkörpers in die Hülse durch ein relatives Zurückbleiben des Reibschlusselementes gegenüber dem Kolbenkörper spannbar ist, wobei durch eine Entspannung des Federelements ein zumindest teilweises Austreiben des Kolbenkörpers aus der Hülse bei unbewegtem Reibschlusselement zur Vergrößerung des Hülseninnenraums bewirkbar ist.

Zudem betrifft die vorliegende Erfindung ein Reibschlussfederelement umfassend ein Reibschluss- und ein Federelement für eine Spritze wie oben beschrieben.

Das erfindungsgemäße Reibschlussfederelement weist in einer vorteilhaften Ausführungsform einen Profilring mit einem im Wesentlichen H-förmigen Querschnitt auf, wobei der außenliegende Profilschenkel das Reibschlusselement und der Profilquerschenkel und der innenliegende Profilschenkel das Federelement bilden, wobei der innenliegende Profilschenkel zum Anliegen an eine Außenfläche eines Kolbenkörpers ausgestaltet ist (siehe beispielsweise Fig.1c, 2c, 2d, 3b, 4b, 9c oder 15c).

Bei einem Profilring mit im Wesentlichen H-förmigem Querschnitt ergibt sich eine geeignete Auflagefläche des außen liegenden Profilschenkels, also des Reibschlusselementes, an einer Innenwandung der Hülse, wobei durch den innen liegenden Profilschenkel ein Aufliegen des Federelementes auf einer Außenfläche des Kolbenkörpers gewährleistet ist, wobei wiederum insbesondere der Profilquerschenkel des Querschnitts eine Scherung des Reibschlussfederelementes zum Spannen des Federelementes erlaubt.

In einer bevorzugten Modifikation der oben beschriebenen vorteilhaften Ausführungsform mit im Wesentlichen H-förmigem Querschnitt weist der innenliegende Profilschenkel (als Teil des Federelements) eine geringere Ausdehnung in Längsrichtung der Spritze auf als der außenliegende Profilschenkel (als Reibschlusselement). Bei einem derartig verlängerten bzw. vergrößerten außenliegenden Profilschenkel ergibt sich der Vorteil, das die durch den außenliegenden Profilschenkel gebildete Dichtlippe durch den Mediendruck, der sich beim Applizieren, insbesondere im Fall einer engen oder sehr engen Kanüle, ergibt, über einen großen Bereich an die Hülseninnenwand gedrückt wird, womit sich eine besonders effektive Dichtwirkung erreichen lässt.

In einer weiteren vorteilhaften Modifikation weist der Profilquerschenkel des Reibschlussfederelements, der als Teil des Federelements den innenliegenden Profilschenkel und den außenliegenden Profilschenkel verbindet, eine in radialer Richtung nach außen abnehmende Dicke in Längsrichtung der Spritze auf. Mit anderen Worten nimmt bei dieser Modifikation die Stärke des Profilquerschenkels zwischen dem innenliegenden Profilschenkel und dem außenliegenden Profilschenkel von außen nach innen zu, wobei sich der Begriff "Stärke" hier jeweils auf den Abstand zwischen der dem Hülseninnenraum zugewandten Randfläche zu der vom Hülseninnenraum abgewandten Randfläche des Profilquerschenkels bezieht. Mit dem Vorsehen einer derartigen Verjüngung des Profilquerschenkels nach außen hin kann eine gewünschte Reduktion der Rückstellkraft erreicht werden, die je nach Viskosität des abzugebenden Material wünschenswert sein kann, ohne dass ein insgesamt sehr dünner Profilquerschenkel hergestellt werden müsste. Insbesondere bei einer Herstellung per Spritzguss kann bei einem Profilquerschenkel, der über einen vergleichsweise großen Anteil einen kleinen Querschnitt besitzt, die Gefahr von Produktionsfehlern in Form einer fehlerhaften Ausfüllung ansteigen.

In einer alternativen vorteilhaften Ausführungsform der vorliegenden Erfindung umfasst das Reibschlussfederelement einen Profilring mit einem im Wesentlichen T-förmigen Querschnitt, wobei der außenliegende Profilschenkel (der dem waagerechten Balken beim Buchstaben "T" entspricht) das Reibschlusselement und der Profilquerschenkel (der dem senkrechten Balken beim Buchstaben "T" entspricht) das Federelement bildet, wobei das Federelement zum Einspannen in einem Kolbenkörper ausgestaltet ist (siehe beispielsweise Fig. 10c). Eine weitere Alternative besteht in einem im Wesentlichen L-förmigen Querschnitt, wobei sich der Profilquerschenkel bei L-förmigem Querschnitt an einem Ende des außenliegenden Profilschenkels und nicht wie bei T-förmigem Querschnitt im Mittelbereich des außenliegenden Profilschenkels an diesen anschließt.

Ist das Reibschlussfederelement in Form eines Profilrings mit im Wesentlichen T-förmigem Querschnitt ausgebildet, weist dieses Reibschlusselement eine mittlere Öffnung auf, durch die sich ein erstes beziehungsweise zweites Teil des Kolbenkörpers zu einer Fixierung beziehungsweise Verankerung des Reibschlussfederelementes erstrecken kann, wobei das Reibschlussfederelement durch die Verbindung von erstem und zweiten Teil des Kolbenkörpers sowohl kraft- als auch formschlüssig gehalten werden kann. Insbesondere der querliegende Profilschenkel bildet das Federelement, das hierbei auf einfache Weise eingespannt werden kann und wiederum eine Scher- beziehungsweise Biegekraft erfährt, wenn das Federelement gespannt wird. Der außen liegende Profilschenkel des Reibschlussfederelementes übernimmt die Funktion des Reibschlusselementes.

In noch einer alternativen Ausführungsform umfasst das erfindungsgemäße Reibschlussfederelement einen im Wesentlichen tellerförmigen Bereich, wobei der Rand des tellerförmigen Bereichs das Reibschlusselement und der Mittelbereich des tellerförmigen Bereichs das Federelement bildet (siehe beispielsweise 5c, 6c oder 7c).

Das Reibschlussfederelement kann auch teller- oder scheibenförmig ausgestaltet sein, wobei der äußere Bereich beziehungsweise Rand des Reibschlussfederelementes die Funktion des Reibschlusselementes, d. h. das Anliegen an der Innenwandung einer Hülse und das Bilden eines Reibschlusses erfüllt, wobei durch eine Verformung des Innen- beziehungsweise Mittelbereichs des Tellerbeziehungsweise scheibenförmigen Reibschlussfederelementes eine Spannung des Federelementes erreicht wird.

Bevorzugt weist gemäß einer vorteilhaften Ausführungsform der Erfindung das Reibschlusselement des Reibschlussfederelements einen Vorsprung auf, der zum Eingriff in eine Einbuchtung eines Kolbenkörpers ausgestaltet ist.

Ist das Reibschlusselement des Reibschlussfederelementes mit einem Vorsprung versehen, der in eine Einbuchtung des Kolbenkörpers eingreifen kann, kann damit eine relative Fixierung zwischen Kolbenkörper und Reibschlussfederelement erreicht werden, wobei die Größe beziehungsweise Ausgestaltung der Einbuchtung dennoch eine Relativbewegung zwischen zumindest Reibschlusselement und Kolbenkörper erlaubt.

Bei den obigen Bezugnahmen auf die beiliegenden Figuren sind die Figuren jeweils nur als beispielhafte Illustrierung einzelner Aspekte gedacht. Die Figuren sind hierzu nicht als Ein- oder Beschränkung zu verstehen. Die obigen Bezugnahmen dienen allein dem besseren Verständnis.

Im Folgenden wird die Erfindung anhand bevorzugter Ausführungsbeispiele unter Bezugnahme auf die beiliegenden Figuren näher erläutert. Hierbei zeigt:
- Fig. 1 a, 1b, 1 c: jeweils ein erstes Ausführungsbeispiel einer erfindungsge- mäßen Spritze in Ansicht, Querschnitt und Ausschnittsverg- rößerung in einem Zustand mit ungespanntem Federele- ment,
- Fig. 2a, 2b, 2c, 2d: jeweils das erste Ausführungsbeispiel der erfindungsge- mäßen Spritze aus den Fig. 1a, 1b und 1c in Ansicht, Querschnitt, Ausschnittsvergrößerung und weiterer Ver- größerung in einem Zustand mit gespanntem Federele- ment,
- Fig. 3a, 3b: jeweils eine Draufsicht und eine Querschnittsansicht eines ersten Ausführungsbeispiels eines erfindungsgemäßen Reibschlussfederelements ähnlich dem des ersten Ausfüh- rungsbeispiels der erfindungsgemäßen Spritze in unges- panntem Zustand,
- Fig. 4a, 4b: jeweils eine Draufsicht und eine Querschnittsansicht des ersten Ausführungsbeispiel eines erfindungsgemäßen Reibschlussfederelements aus den Fig. 3a und 3b in ge- spanntem Zustand,
- Fig. 5a, 5b, 5c: jeweils ein zweites Ausführungsbeispiel einer erfindungs- gemäßen Spritze in Ansicht, Querschnitt und Ausschnitt- svergrößerung in einem Zustand mit ungespanntem Feder- element,
- Fig. 6a, 6b, 6c: jeweils das zweite Ausführungsbeispiel der erfindungsge- mäßen Spritze aus den Fig. 5a, 5b und 5c in Ansicht, Querschnitt und Ausschnittsvergrößerung in einem Zustand mit gespanntem Federelement,
- Fig. 7a, 7b, 7c: jeweils ein drittes Ausführungsbeispiel einer erfindungsge- mäßen Spritze in Ansicht, Querschnitt und Ausschnittsverg- rößerung in einem Zustand mit ungespanntem Federele- ment,
- Fig. 8a, 8b, 8c, 8d: jeweils ein zweites Ausführungsbeispiele eines erfindungs- gemäßen Reibschlussfederelements in schematischer An- sicht, erstem Querschnitt, zweitem Querschnitt und in perspektivischer Ansicht,
- Fig. 9a, 9b, 9c: jeweils ein viertes Ausführungsbeispiel einer erfindungs- gemäßen Spritze in Ansicht, Querschnitt und Ausschnitt- svergrößerung in einem Zustand mit gespanntem Feder- element,
- Fig. 10a, 10b, 10c: jeweils ein fünftes Ausführungsbeispiel einer erfindungs- gemäßen Spritze in Ansicht, Querschnitt und Ausschnitt- svergrößerung in einem Zustand mit ungespanntem Feder- element,
- Fig. 11: eine perspektivische Explosionsdarstellung eines Ausführungsbeispiels eines erfindungsgemäßen Kolbens, wie er im in den Fig. 10a, 10b und 10c gezeigten Ausfüh- rungsbeispiele einer Spritze vorgesehen ist,
- Fig. 12: ein schematisches Ablaufdiagramm eines Ausführungsbei- spiels eines erfindungsgemäßen Verfahrens,
- Fig. 13a, 13b, 13c: jeweils ein sechstes Ausführungsbeispiel einer erfindungs- gemäßen Spritze in Ansicht, Querschnitt und Ausschnitts- vergrößerung in einem Zustand mit ungespanntem Feder- element,
- Fig. 14: eine perspektivische Ansicht eines Ausführungsbeispiels einer erfindungsgemäßen Spritze,
- Fig. 15a, 15b, 15c: jeweils ein siebtes Ausführungsbeispiel einer erfindungs- gemäßen Spritze in Ansicht, Querschnitt und Ausschnitts- vergrößerung in einem Zustand mit ungespanntem Feder- element, und
- Fig. 16a, 16b, 16c: jeweils das siebte Ausführungsbeispiel einer erfindungs- gemäßen Spritze in Ansicht, Querschnitt und Ausschnitts- vergrößerung ähnlich zu den Fig. 15a, 15b und 15c in ei- nem Zustand mit gespanntem Federelement.

Ähnliche bzw. einander entsprechende Elemente sind in den Figuren mit gleichen Bezugzeichen gekennzeichnet, auch wenn die Element in unterschiedlichen Ausführungsbeispielen unterschiedlich ausgestaltet sind.

Fig. 1a, 1b, 1c zeigen jeweils ein erstes Ausführungsbeispiel einer erfindungsgemäßen Spritze in Ansicht, Querschnitt und Ausschnittsvergrößerung in einem Zustand mit ungespanntem Federelement. Fig. 1a zeigt eine erfindungsgemäße Spritze gemäß einem ersten Ausführungsbeispiel in einer unbelasteten Stellung. Fig. 1b ist eine Schnittdarstellung entlang des gezeichneten Schnittverlaufes. Fig. 1c zeigt den Dichtungsbereich bzw. den Bereich des Reibschlussfederelements in einer vergrößerten Darstellung.

Fig. 1a zeigt die Spritze 1 gemäß dem ersten Ausführungsbeispiel. Die Spritze 1 umfasst eine Hülse 10 und einen Kolben 15, von dem in Fig. 1 nur der Kolbenkörper zu sehen ist. Die Hülse 10 weist an ihrem der Auslassöffnung gegenüberliegenden Ende einen Griff 45 auf, der eine Bedienung der Spritze 1 bei Eindrücken des Kolbens 15 erleichtert. Das der Auslassöffnung gegenüberliegende Ende dient zur Aufnahme des Kolbens 15.

In Fig. 1b ist die Spritze 1 in einer Querschnittsansicht gezeigt. Die Spritze 1 weist die Hülse 10 mit einer Innenwandung 35, einer Auslassöffnung 40 und dem Griff 45 auf. In die Hülse 10 eingebracht ist der Kolben mit dem Kolbenkörper 15' und dem Reibschlussfederelement 20, einer einstückigen Kombination aus Reibschlusselement und Federelement. Der Kolbenkörper 15' besitzt eine Spitze 75, die zu der Auslassöffnung 40 räumlich angepasst ist. Die Hülse 10 schließt einen Hülseninnenraum 70 ein, der an einer der Auslassöffnung 40 gegenüberliegenden Seite von dem Kolben, d.h. von Kolbenkörper 15' (bzw. Kolbenkörperspitze 75) und Reibschlussfederelement begrenzt wird.

Fig. 1c zeigt eine vergrößerte Darstellung des Bereich um das Reibschlussfederelement 20 aus Fig. 1b. Das Reibschlussfederelement 20 wird durch ein Ringprofil (hier nur im Querschnitt gezeigt) gebildet, das ein Federelement 30 und ein Reibschlusselement 25 umfasst, wobei das Reibschlussfederelement 20 einen H-förmigen Querschnitt aufweist.

Das Reibschlusselement 25 liegt reibschlüssig an der Innenwandung 35 der Hülse 10 an und bildet somit eine Dichtung gegenüber einem Durchtritt von Werkstoff aus dem Hülseninnenraum 70 zwischen Reibschlusselement 25 und Innenwandung 35.

Das Federelement 30, gebildet vom Querschenkel und Innenschenkel des Profilrings, ist in einer Ausnehmung 50 des Kolbenkörpers 15' aufgenommen, wobei die Ausnehmung angepasst ist, den Innenschenkel des Profilrings anliegend aufzunehmen, womit sich eine Dichtwirkung zwischen Profilring und Ausnehmung ergibt.

In den Fig. 1a, 1b und 1c ist das Federelement 30 in ungespanntem Zustand dargestellt.

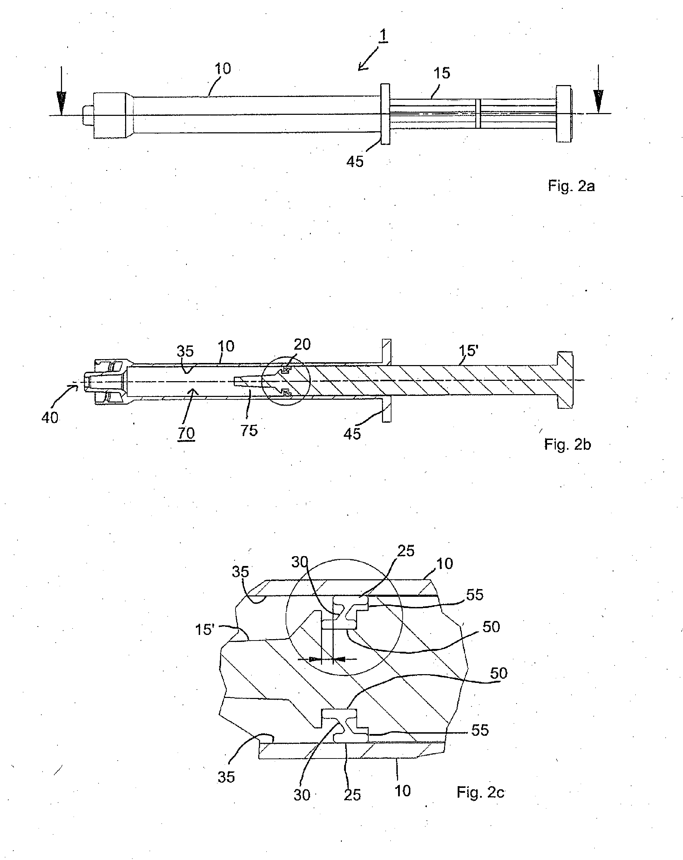

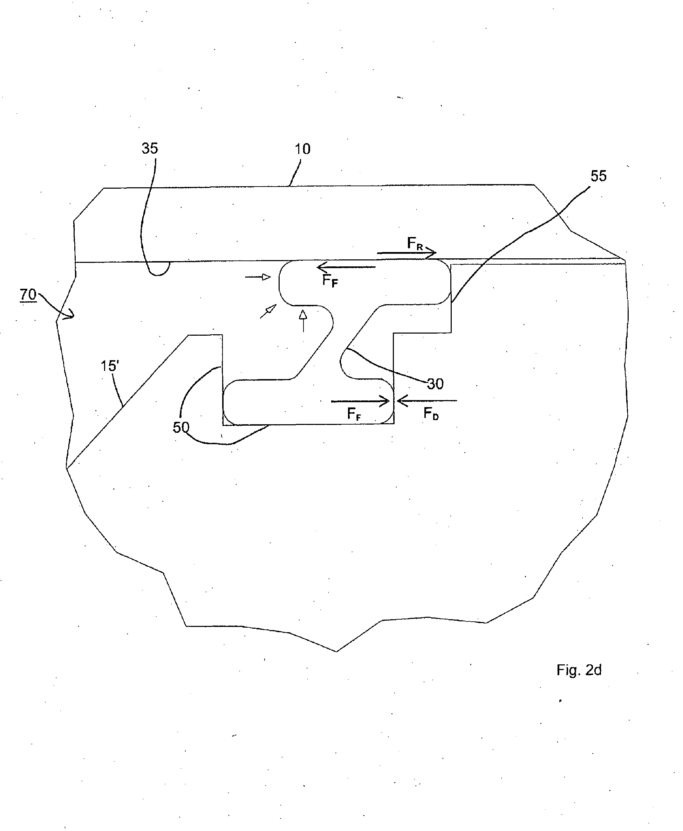

Fig. 2a, 2b, 2c, 2d zeigen jeweils das erste Ausführungsbeispiel der erfindungsgemäßen Spritze aus den Fig. 1a, 1b und 1c in Ansicht, Querschnitt, Ausschnittsvergrößerung und weiterer Vergrößerung in einem Zustand mit gespanntem Federelement. Fig. 2a zeigt Spritze aus den Fig. 1a, 1b und 1c in einer Position während der Abgabe von Material, d.h. mit gespanntem Federelement. In der Schnittdarstellung in Fig. 2b und besser noch in der Vergrößerung des Dichtungsbereiches in Fig. 2c ist zu sehen, wie sich Federelement 30 der Dichtung bzw. des Reibschlussfederelements 20 elastisch verformt hat. Eine noch größer dargestellte Ansicht einer Seite der Dichtung ist in Fig. 2d abgebildet.

Die Fig. 2a und 2b entsprechen weitgehend der Darstellung des ersten Ausführungsbeispiels in den Fig. 1a und 1 b. Der Unterschied besteht darin, dass das Federelement 30 hierbei gespannt ist.

In Fig. 2c ist deutlicher zu erkennen, dass das Federelement 30 durch Einwirken einer Scherkraft gespannt ist, wobei ein Versatz zwischen dem Innenschenkel und dem Außenschenkel des Profilrings besteht. Diese Scherung bzw. Spannung ist durch ein Einbringen des Kolben(körper)s in die Hülse bewirkt, bei der das Reibschlusselement 25 infolge der wirkenden Reibungskraft dem Eindringen des Kolbenkörpers 15' nicht vollständig folgt. Da das Federelement 30 mit seinem Profilinnenschenkel in der Ausnehmung 50 fest am Kolbenkörper 15' verankert ist, folgt damit eine relative Bewegung zwischen Reibschlusselement 25 und Profilinnenschenkel (bzw. Kolbenkörper 15'). Diese relative Bewegung führt zum Spannen des Federelements 30.

In Fig. 2c ist zudem zu erkennen, dass das Reibschlusselement 25 an eine Kante 55 des Kolbenkörpers 15' anstößt. Auch bei einem weiteren Bewegen des Kolbenkörpers 15' bzw. Kolbens 15 in die Hülse 10 (nach links in den Figuren) würde durch das Mitnehmen des Reibschlusselements 25 über die Kante 55 eine weitere Spannung des Federelements, bei der größere Federkräfte aufträten, verhindert. Eine Verformung des Reibschlussfederelement wird durch die Kante 55 begrenzt. Hierdurch kann insbesondere vermieden werden, dass das Reibschlussfederelement überdehnt würde und sogar reißen könnte.

Wie in besonders Fig. 2d zu erkennen ist, weist das Reibschlusselement 30 eine in Richtung des Hülseninnenraums 70 ragende Lippe auf. Wie durch die kleinen Pfeile in Fig. 2d angedeutet, wirkt ein Druck des Werkstoffs, der sich in dem Hülseninnenraum 70 befindet, auf diese Lippe, was die Dichtwirkung des Reibschlusselements 30 verbessert. Eine Abdichtung zur inneren Mantelfläche des Spritzenkörpers, also der Innenwandung 35 der Hülse 10, wird unterstützt durch die lippenförmig ausgestaltete umlaufende Kante des Reibschlusselements 25, auf die bei einem flüssigen oder fließfähigen Werkstoff ein Flüssigkeitsdruck wirkt.

In Fig. 2d sind zudem Kraftpfeile angedeutet, die wirkende Kräfte vereinfachend symbolisieren. Eine Druckkraft F_{D} wirkt zunächst vom Kolbenkörper 15' auf das gespannte Federelement 30. Das gespannte Federelement übt eine Kraft F_{F} auf den Kolbenkörper 15' aus. Im hier betrachteten Gleichgewichtsfall sind die Kräfte gleich. Die Kraft F_{F} des Federelements 30 wirkt allerdings auch auf das Reibschlusselement 25 bzw. über das Reibschlusselement 25 an der Grenzfläche zur Innenwandung 35. Zwischen Innenwandung 35 und Reibschlusselement 25 wirkt auch die dieser Federkraft F_{F} entgegengesetzte Reibkraft F_{R}. Wiederum ist im vorliegenden Fall die Reibkraft F_{R} gleich der Federkraft F_{F}.

Für den vorliegenden Gleichgewichtsfall ist es ohne Belang, ob der Kolben aktuell in die Hülse eingeführt wird oder ob der Kolben relativ zur Hülse ruht. Ein Unterschied zwischen Einführen und Ruhen besteht allenfalls in der Größe der Reibungskraft, also einem eventuellen Unterschied zwischen Haftreibung und Gleitreibung zwischen Innenwandung 35 und Reibschlusselement 25.

Das Gleichgewicht der Kräfte führt (innerhalb gewisser Grenzen, die hier durch u.a. durch die Kante 55 gegeben sind) zu einer bestimmten Ausrichtung der Elemente zueinander. Wäre die wirkende Druckkraft größer als die dargestellte Kraft F_{D}, so würde dies - solange kein Anstoßen des Reibschlusselements an der Kante 55 erfolgt - zu einer größeren Spannung bzw. Verformung des Federelements 30 führen, was wiederum ein entsprechende Auswirkung auf die Kraftverhältnisse an der Grenzfläche zwischen Innenwandung 35 und Reibschlusselement 25 hätte. Solange die Federkraft F_{F} kleiner oder gleich der Reibkraft F_{R} ist, wird die Auslenkung bzw. Verspannung durch die Reibkraft gehalten bzw. vergrößert. Ist die Federkraft größer als die Reibkraft (etwa bei einem Übergang von Haftreibung zu Gleitreibung), so wird sich die Verformung des Federelements 30 zurückbilden, bis die (damit sich verringernde) Federkraft und die Reibkraft wieder im Gleichgewicht sind. Durch die Kante 55 ist eine Begrenzung bzw. Limitierung der Verschiebung zwischen Reibschlusselement 25 und (Innenschenkel des) Federelement(s) 30 bzw. der Spannung des Federelements 30 gegeben. Sobald das Reibschlusselement 25 an der Kante 55 anstößt, wird direkt über die Kante 55 und nicht mehr nur über das Federelement 30 Kraft in das Reibschlusselement 25 eingeleitet, sodass die Reibkraft auch dann überwunden werden kann, wenn die Federkraft allein dazu nicht ausreicht. Anders gesagt, wird der Aufbau einer Federkraft auf einen bestimmten Maximalwert begrenzt, da die Kante 55 (bzw. der Kolbenkörper 15') ab dem Anstoßen auf das Reibschlussfederelement als Ganzes und nicht mehr nur auf das Federelement 30 Kraft wirken lässt, sodass eine weitere Verformung bzw. Spannung des Reibschlussfederelements ausbleibt.

Fig. 3a, 3b zeigen jeweils eine Draufsicht und eine Querschnittsansicht eines ersten Ausführungsbeispiels eines erfindungsgemäßen Reibschlussfederelements ähnlich dem des ersten Ausführungsbeispiels der erfindungsgemäßen Spritze in ungespanntem Zustand.

Fig. 3a und Fig. 3b zeigen ein erfindungsgemäßes Reibschlussfederelement 20, das auch als Dichtung verwendet werden kann, in einem unbelasteten, also ungespannten, Zustand. Das Reibschlussfederelement 20 besteht aus einem einstückigen Profilring, der einen H-förmigen Querschnitt aufweist. Der Außenschenkel des H bildet hierbei das Reibschlusselement 25, während der Innenschenkel und der Querschenkel zusammen das Federelement 30 bilden. Das Reibschlussfederelement 20 weist eine symmetrische Ausgestaltung auf. Dies stellt für den Montagevorgang, also für den Zusammenbau der erfindungsgemäßen Spritze, eine Erleichterung dar. Vorder- und Rückseite sind identisch ausgestaltet, womit das Reibschlussfederelement 20 unabhängig von einer Orientierung auf den Kolbenkörper aufgesetzt werden kann. Bei einer solcher Ausgestaltung ist die Fehlerquelle eines fehlerhaften, weil falsch orientierten, Zusammenbau ausgeschlossen.

Fig. 4a, 4b zeigen jeweils eine Draufsicht und eine Querschnittsansicht des ersten Ausführungsbeispiels eines erfindungsgemäßen Reibschlussfederelements aus den Fig. 3a und 3b in gespanntem Zustand. Das Reibschlussfederelement 20 befindet sich in einem verformten Zustand, bei dem das Reibschlusselement 25 gegenüber seinem Ruhezustand und insbesondere gegenüber dem Innenschenkel des Federelements 30 entlang einer Achse senkrecht zur Ebene des Reibschlussfederelements 20 verschoben ist. Mit dieser Verschiebung bzw. Verformung ist das Federelement 30 unter Spannung gesetzt, wobei das Reibschlussfederelement 20 Kräfte ausübt, um wieder in den ungespannten Ruhezustand zurückzukehren. Im eingebauten Zustand werden diese Kräfte einerseits über die Reibung zwischen dem Reibschlusselement 25 und der Innenwandung einer Hülse, an der das Reibschlusselement 25 anliegt, aufgenommen und andererseits auf den Kolbenkörper übertragen, an dem das Reibschlusselement 20, insbesondere über den Innenschenkel, angebracht ist. Über eine Krafteinwirkung durch den Kolbenkörper parallel zu der Mittelachse des Reibschlussfederelements 20 einerseits und die Reibung an der Innenwandung andererseits erfolgt auch die dargestellte Verformung des Reibschlussfederelements 20 zu seiner Spannung.

Fig. 5a, 5b, 5c zeigen jeweils ein zweites Ausführungsbeispiel einer erfindungsgemäßen Spritze in Ansicht, Querschnitt und Ausschnittsvergrößerung in einem Zustand mit ungespanntem Federelement.

Die Spritze 1 unterscheidet sich von der in den Figuren 1a und 2a dargestellten Spritze zunächst in der Anschlussmöglichkeit an der Auslassöffnung, bei der vorliegend ein Außengewinde im Gegensatz zu einem Innengewinde dargestellt ist. Die Befestigung etwa einer Kanüle wird in diesem Fall über das Außengewinde vorgenommen, kann aber in anderen Ausführungsbeispielen auch anders erfolgen. Einige Möglichkeiten sind z.B. ein Bajonettverschluss oder ein Konus mit Selbsthemmung.

Zudem ist die Auslassöffnung 40 im Wesentlichen flach ausgestaltet. Der wesentliche Unterschied zwischen der Spritze nach dem ersten Ausführungsbeispiel und der Spritze 1 nach dem zweiten Ausführungsbeispiel besteht in der Ausgestaltung des Reibschlusselements 20 und des Kolbenkörpers 15', ansonsten entsprechen sich die ersten und zweiten Ausführungsbeispiele.

Das Reibschlusselement 20 weist auf seiner dem Hülseninnenraum 70 zugewandten Seite eine im Wesentlichen flache Oberfläche auf, die von einem scheibenförmigen Federelement 30 gebildet wird. An der Außenseite des Federelements 30 schließt sich das Reibschlusselement 25 an, dass das Federelement 30 und die Innenwandung 35 der Hülse 10 voneinander beabstandet. Das Reibschlusselement 30 liegt reibschlüssig an der Innenwandung 35 an und erstreckt sich im Wesentlichen von dem Hülseninnenraum 35 weg, der von Kolben 15 und Hülse 10 definiert wird. Das Reibschlusselement 35 weist hierbei einen Vorsprung 65 auf, der sich in Richtung von der Innenwandung 35 weg nach innen erstreckt.

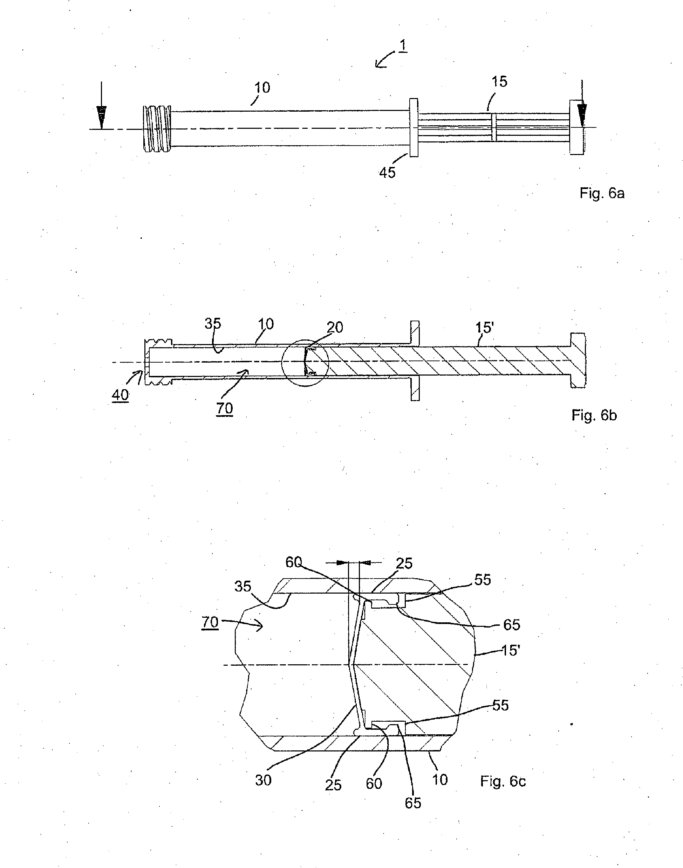

Der Kolbenkörper 15' weist an seinem vorderen, dem Hülseninnenraum 70 zugewandten Ende eine flach zulaufende Spitze auf, deren Funktion weiter unten mit Bezug auf die Figuren 6a, 6b und 6c beschrieben wird. In seinem vorderen Bereich weist der Kolbenkörper 15' einen umlaufenden, nach außen (d.h. in Richtung auf die Hülseninnenwandung 35) gerichteten Vorsprung 60 auf. Wie auch im ersten Ausführungsbeispiel besitzt der Kolbenkörper zudem eine Kante 55, wobei sich zwischen der Kante 55 und dem Vorsprung somit eine Ausnehmung in Kolbenkörper 15' ergibt, in die sich der Vorsprung 65 des Reibschlusselements 25 bzw. des Reibschlussfederelements 20 erstreckt. Der Vorsprung 65 des Reibschlusselements 25 und damit das Reibschlusselement selbst werden durch den Vorsprung 60 und die Kante 55 des Kolbenkörpers 15' im Zusammenspiel mit der Hülseninnenwandung 35 fixiert, wobei die Größe (d.h. die Erstreckung) der Ausnehmung (in Längsrichtung) ein Spiel für das Reibschlusselement 25 bestimmt, innerhalb dessen eine Bewegung relativ zum Kolbenkörper 15' trotz der Fixierung möglich ist. Die Bewegungsmöglichkeit ergibt sich entweder mit dem Abstand zwischen der Kante 55 und dem Vorsprung 65 (bei entsprechend großen bzw. langem Reibschlusselement) oder mit dem Abstand des Vorsprungs 65 vom Anschluss des Federelements 30 am Reibschlusselement 25 wie in der Darstellung in Fig. 5c (siehe auch Fig. 6c).

Gemäß dem zweiten Ausführungsbeispiel ist das Reibschlussfederelement 20 durchgehend und trennt den Kolbenkörper 15' vom Hülseninnenraum. Mit einem annähernd flachen Kolbenkörper mit einer stumpfen Spitze bei einer kurzen oder flachen Auslassöffnung 40 der Spritze kann der in der Spritze enthaltene Werkstoff vollständig abgegeben werden. Auch beim Befüllen ergibt sich damit kein bzw. ein nur sehr geringer Lufteinschluss im Inneren der Spritze.

Durch das Ineinandergreifen der Vorsprünge 60 und 65 des Kolbenkörpers 15' und des Reibschlusselements 25 ist auch ein Zurückziehen des Kolbens 15 möglich, ohne dass sich das Reibschlussfederelement 20 dabei vom Kolbenkörper 15' löst. Ein solches Zurückziehen kann insbesondere bei einem Befüllen der Spritze 1 durch die Auslassöffnung hindurch vorgesehen sein, wobei durch das Zurückziehen im Hülseninnenraum 70 ein Unterdruck erzeugt wird, um Werkstoff beispielsweise in Form einer Flüssigkeit einzusaugen.

Gegenüber dem Aufziehen des Reibschlussfederelements 20 auf den Kolbenkörper 15 (d.h. in die Ausnehmung 50), wie es bei dem ersten Ausführungsbeispiel zur Montage vorgesehen ist, weist das zweite Ausführungsbeispiel den Vorteil einer einfacheren Montage durch ein Aufsetzen des Reibschlusselements 20 auf die Spitze des Kolbenkörpers 15' auf. Hierbei ist es allerdings bei dem gezeigten Ausführungsbeispiel infolge der Asymmetrie des Reibschlussfederelements 20 nötig, dass es in der richtigen Ausrichtung aufgesetzt wird.

Fig. 6a, 6b, 6c zeigen jeweils das zweite Ausführungsbeispiel der erfindungsgemäßen Spritze aus den Fig. 5a, 5b und 5c in Ansicht, Querschnitt und Ausschnittsvergrößerung in einem Zustand mit gespanntem Federelement.

Wie besonders in Fig. 6c zu erkennen ist, befindet sich das Federelement 30 vorliegend in gespanntem Zustand. Hierbei ist das Federelement 30 durch das relative Zurückbleiben des Reibschlusselements 25 an die Spitze des Kolbenkörpers 15' angelegt. Diese Spitze hat die Form eines sehr flachen Kegels, damit keine zu starke Verformung des Federelements 30 auftritt. In Fig. 6c ist zudem zu erkennen, dass auch bei an der Spitze anliegendem Federelement 30 das Reibschlusselement 25 nicht an der Kante 55 des Kolbenkörpers 15' anliegt. Die Verformung und damit die Spannung des Federelements 30 wird somit vorliegend durch die Form der Spitze des Kolbenkörpers 15' bestimmt.

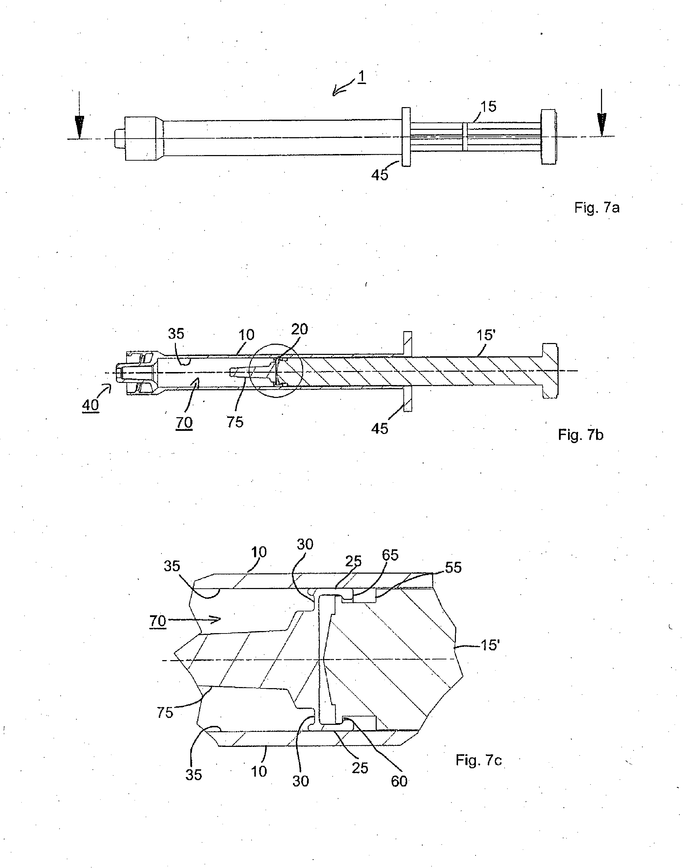

Fig. 7a, 7b, 7c zeigen jeweils ein drittes Ausführungsbeispiel einer erfindungsgemäßen Spritze in Ansicht, Querschnitt und Ausschnittsvergrößerung in einem Zustand mit ungespanntem Federelement.

Das dritte Ausführungsbeispiel unterscheidet sich vom zweiten Ausführungsbeispiel darin, dass - ähnlich zur Spitze 75 des Kolbenkörper im ersten Ausführungsbeispiel - hier das Reibschlussfederelement 20 mit einer Spitze 75 versehen ist, die an die hier ebenfalls vorliegende sich verjüngende Auslassöffnung 40 angepasst ist. Die Spitze kann alternativ zu der gezeigten einstückigen Ausführung auch in geeigneter Weise an einem Reibschlusselement ähnlich dem des zweiten Ausführungsbeispiels angebracht sein. Die obigen Ausführungen zum zweiten Ausführungsbeispiel gelten entsprechend auch für das dritte Ausführungsbeispiel

Fig. 8a, 8b, 8c, 8d zeigen jeweils ein zweites Ausführungsbeispiel eines erfindungsgemäßen Reibschlussfederelements in schematischer Ansicht, erstem Querschnitt, zweitem Querschnitt und in perspektivischer Ansicht.

In Fig. 8a ist eine schematische Ansicht eines Reibschlussfederelements 20 gemäß dem zweiten Ausführungsbeispiel gezeigt, wobei das Reibschlussfederelement 20 mit sechs Versteifungselementen 80 versehen ist. Jeweils drei dieser Versteifungselemente befinden sich auf gegenüberliegenden Seiten des Reibschlussfederelements, was in Fig. 8a durch gestrichelte Linien angedeutet ist. Die Figuren 8b und 8c zeigen jeweils Querschnittsansichten des Reibschlussfederelements 20. Abgesehen von den stegförmigen Versteifungselementen oder Verstärkungen 80 entspricht das Reibschlussfederelement 20 dem des ersten Ausführungsbeispiels. Das Reibschlussfederelement 20 ist mit den zusätzlichen stegförmigen Verstärkungen 80 ausgestattet ist, um die Federeigenschaften zu unterstützen. Diese Stege können sich auf einer oder bevorzugt auch auf beiden Seiten befinden, wobei sie wie dargestellt zueinander versetzt sein können. Die Anzahl kann von 1 bis zu einer Vielzahl variieren.

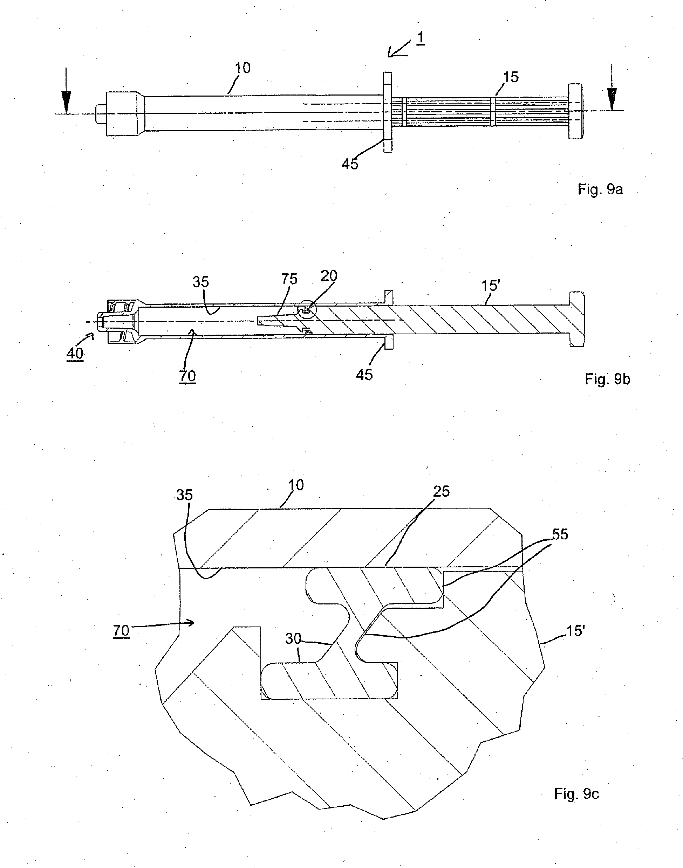

Fig. 9a, 9b, 9c zeigen jeweils ein viertes Ausführungsbeispiel einer erfindungsgemäßen Spritze in Ansicht, Querschnitt und Ausschnittsvergrößerung in einem Zustand mit gespanntem Federelement.

Das vierte Ausführungsbeispiel entspricht wiederum weitgehend dem ersten Ausführungsbeispiel, sodass hier auf eine Beschreibung identischer Merkmale verzichtet werden kann. Anders als beim ersten Ausführungsbeispiel der Spritze ist der Kolbenkörper 15' gemäß dem vierten Ausführungsbeispiel mit einer Kante 55 versehen, an die sich das Reibschlussfederelement 20 sowohl mit seinem Reibschlusselement 25 (wie beim ersten Ausführungsbeispiel) als auch mit dem verformten, d.h. gespannten, Federelement 30 im gespannten Zustand anlegt. Die Kontur der Kante 55 ist dabei so ausgelegt, dass die Kante 55 das Reibschlussfederelement 20 im gespannten Zustand auf der dem Hülseninnenraum 70 abgewandten Seite des Reibschlussfederelements 20 vollflächig stützt. Damit wird eine sichere Begrenzung der Verformung des Reibschlussfederelements über seine gesamte Ausdehnung erreicht, so dass die Gefahr von Beschädigungen, beispielsweise eines Reißens durch Einschneidungen, weitestgehend beseitigt ist. Erfindungsgemäß können jedoch aus Zwischenformen zwischen der Ausgestaltung gemäß erstem und viertem Ausführungsbeispiel hinsichtlich der Kante oder Stufe 55 vorgesehen werden. Mit der Anpassung der Kante 55 an die Form des Reibschlussfederelements ergibt sich im vierten Ausführungsbeispiel ein Hinterschnitt, der eine zusätzliche Haltwirkung für den Fußteil des Federelements (Innenschenkel des H-Profils) hat, der dem Hülseninnenraum abgewandt ist.

Fig. 10a, 10b, 10c zeigen jeweils ein fünftes Ausführungsbeispiel einer erfindungsgemäßen Spritze in Ansicht, Querschnitt und Ausschnittsvergrößerung in einem Zustand mit ungespanntem Federelement.

Gezeigt ist ein erfindungsgemäßes Ausführungsbeispiel, bei dem das Reibschlussfederelement 20 als Dichtung mit einem separaten Kopfstück 85 als erstem Teil des Kolbenkörpers 15' an einem zweiten Teil 90 des Kolbenkörpers 15' befestigt ist. Das Kopfstück 85 ist wiederum geometrisch an die Auslassöffnung 40 angepasst, um das Auftreten von Luft in der Vorderseite der Spritze 1 so weit wie möglich zu minimieren. Mit einer derartigen Anpassung lässt sich die Spritze 1 weitgehend luftfrei von vorne befüllen.

Das Kopfstück 85 ist mit dem zweiten Teil 90 des Kolbenkörpers 15' durch ein Einpressen verbunden. Eine zusätzliche Sicherung gegen eine unerwünschte Trennung von erstem und zweitem Kolbenkörperteil 80, 90 stellt eine Verdickung in Form einer Schnappverbindung dar. Zudem sind weitere Verdickungen in Form von Widerhaken vorhanden, die zwar nicht am Ende einrasten, aber aufgrund der Elastizität des Kunststoffes des zweiten Teils 90 des Kolbenkörpers 15' ebenfalls ein Herausziehen verhindern. Es ist hierbei zweckmäßig, das Kopfstück 85 aus einem festeren Werkstoff als dem des zweiten Teils 90 herzustellen. Somit werden die Widerhaken beim Einpressen weniger stark als die Bohrung des zweiten Teils 90 des Kolbenkörpers 15' verformt.

Das dargestellte Ausführungsbeispiel weist ferner eine umlaufende Kante am Kopfstück 85 auf. Diese Kante dient dazu, das Federelement 30 besser zu halten und die Abdichtung zwischen Federelement 30 und Kopfstück 85 zu verbessern.

Anders als bei den bisherigen Ausführungsbeispielen besitzt das Reibschlussfederelement 20 hier im Querschnittsprofil eine T-Form, wobei der waagerechte Balken des T das Reibschlusselement 25 und der senkrechte Balken des T das Federelement 30 bildet. Auf einen Fuß des Federelements 30 (der innenliegende Balken des H-Profils) kann hier verzichtet werden, da durch das Ineinanderpressen von erstem und zweitem Teil 85, 90 des Kolbenkörpers 15' eine ausreichende Fixierung vorhanden ist. Alternativ oder ergänzend kann jedoch auch ein Reibschlussfederelement mit einem H-Profil wie oben eingesetzt werden, wobei dann eine geeignete Ausnehmung im Kolbenkörper vorzusehen ist, beispielsweise in Form von Einbuchtungen in dem ersten und dem zweiten Kolbenkörperteil.

Fig. 11 zeigt eine perspektivische Explosionsdarstellung eines Ausführungsbeispiels eines erfindungsgemäßen Kolbens, wie er im in den Fig. 10a, 10b und 10c gezeigten Ausführungsbeispiele einer Spritze vorgesehen ist.

In den obigen Ausführungsbeispielen wurden jeweils einstückige Reibschlussfederelemente dargestellt. Es ist allerdings zu verstehen, dass die Erfindung nicht auf solche einstückigen Ausgestaltungen beschränkt ist und auch mehrteilige Elemente, die geeignet kombiniert sind und passend zusammenarbeiten, erfindungsgemäß vorgesehen sein können.

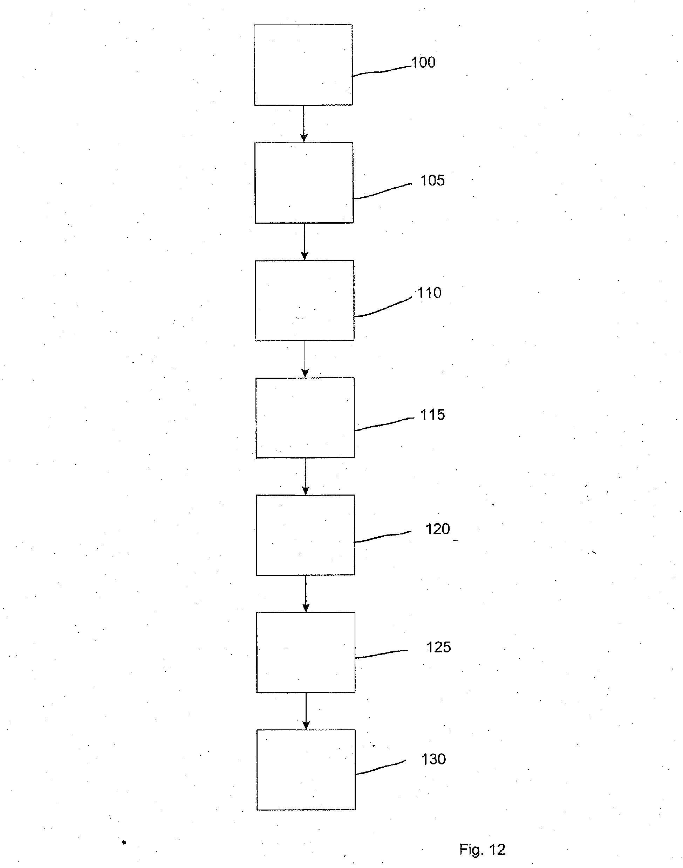

Fig. 12 zeigt ein schematisches Ablaufdiagramm eines Ausführungsbeispiels eines erfindungsgemäßen Verfahrens.

Auf ein Einbringen eines Kolbens mit einem Kolbenkörper, einem Federelement und einem Reibschlusselement in eine Hülse zur Aufnahme von Werkstoff in Schritt 100 folgt ein Bereitstellen von Werkstoff, beispielsweise an der Auslassöffnung der Hülse, in Schritt 105 und ein Ausbringen des Kolbens in Schritt 110. In einer möglichen Anwendung des erfindungsgemäßen Verfahren wird durch das Ausbringen in Form eines Zurückziehens im Inneren der Hülse ein Unterdruck aufgebaut, der ein Füllen der Spritze, d.h. der Hülse, mit Werkstoff veranlasst. Alternativ oder ergänzend kann der Kolben auch durch ein Austreiben über in die Spritze eingebrachten Werkstoff ausgebracht werden.

Anstatt des Aufziehens mittels Unterdruck durch Herausziehen des Kolbens kann die Spritze gleichsam durch einen Überdruck der abzufüllenden Substanz gefüllt werden. Hierbei würde der Kolben durch die in die Kammer einströmende Flüssigkeit/Paste bis zur gewünschten Füllmenge heraus gedrückt werden. Dies ermöglicht auf einfache Weise eine industrielle Füllung von erfindungsgemäßen Spritzen in hohen Stückzahlen.

Somit ist eine mit Werkstoff gefüllte Spritze bereitgestellt. In Schritt 115 wird der Kolben der Spritze mit einer Kraft beaufschlagt, also in die Hülse eingedrückt, sodass der Kolben in die Hülse eindringt. Hierbei wird in Schritt 120 das Federelement gespannt, da zunächst das Reibschlusselement aufgrund der wirkenden Haftreibung unbewegt verbleibt. Im weiteren Verlauf des Einbringens in die Hülse kann auch das Reibschlusselement mitgenommen werden, wobei allerdings noch immer eine Spannung des Federelements durch die Wirkung der Gleitreibung aufrechterhalten bleibt. Wird in Schritt 125 die Kraftbeaufschlagung beendet, so entspannt sich das Federelement in Schritt 130 durch ein Austreiben des Kolbenkörpers, was eine Vergrößerung des Hülseninnenraums zur Folge hat. Durch den dabei resultierenden Saugeffekt wird ein weiteres und unerwünschtes Austreten von Werkstoff aus der Hülse bis zu einer erneuten Kraftbeaufschlagung unterbunden.

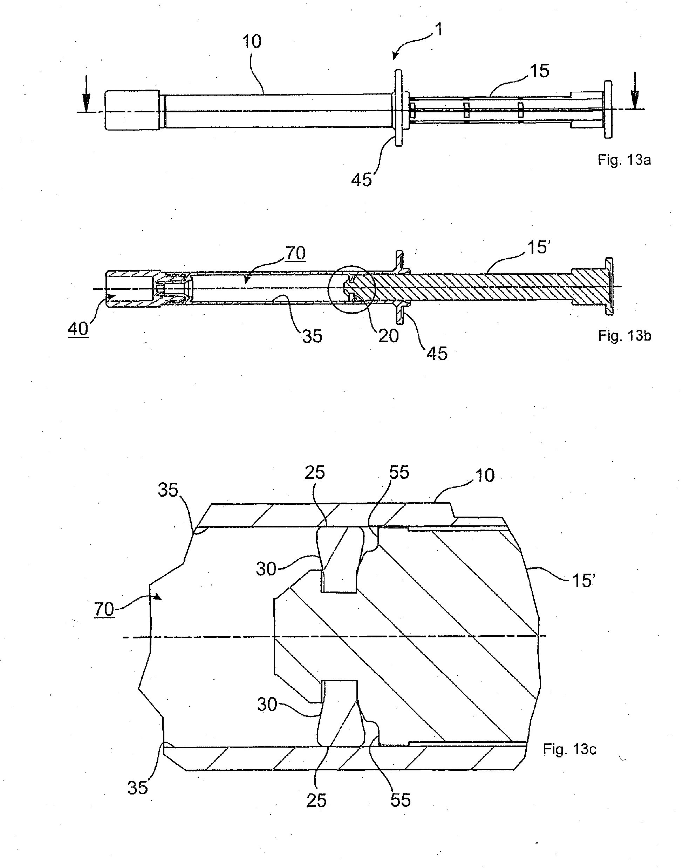

Fig. 13a, 13b, 13c zeigen jeweils ein sechstes Ausführungsbeispiel einer erfindungsgemäßen Spritze in Ansicht, Querschnitt und Ausschnittsvergrößerung in einem Zustand mit ungespanntem Federelement.

Das sechste Ausführungsbeispiel entspricht wiederum weitgehend dem ersten Ausführungsbeispiel, sodass hier auf eine Beschreibung identischer Merkmale verzichtet werden kann. Die Form der Spitze des Kolbenkörpers 15' des sechsten Ausführungsbeispiels der Spritze unterscheidet sich von der des ersten Ausführungsbeispiels, zudem ist die Auslassöffnung 40 mit einem in das Innengewinde der Auslassöffnung 40 eingedrehten Aufsatz versehen. Der Unterschied zwischen dem ersten Ausführungsbeispiel und dem sechsten Ausführungsbeispiel hinsichtlich des Reibschlussfederelements 20 besteht darin, dass beim sechsten Ausführungsbeispiel das Federelement 30 in Relation zum Reibschlusselement 25 im Querschnitt dicker ausgestaltet ist. Mit einem mit stärkerem Querschnitt ausgestalteten Federelement kann bei unveränderter Materialwahl eine größere Steifigkeit bzw. Federkraft eingestellt werden, ähnlich zu den in Figuren 8a bis 8d gezeigten Versteifungselementen.

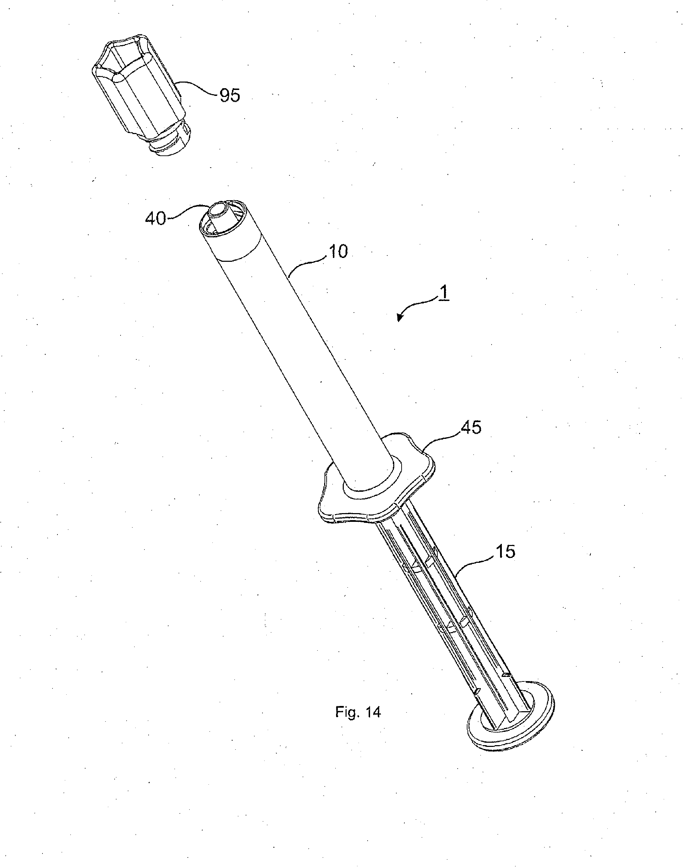

Fig. 14 zeigt eine perspektivische Ansicht eines Ausführungsbeispiels einer erfindungsgemäßen Spritze 1 mit einem Kolben 15 und einer Hülse 10 mit einem Griff 45. Die Auslassöffnung 40 ist mit einem Innengewinde versehen, in das beispielsweise der gezeigte Aufsatz 95 mit der Funktion einer Verschlusskappe eingeschraubt werden kann.

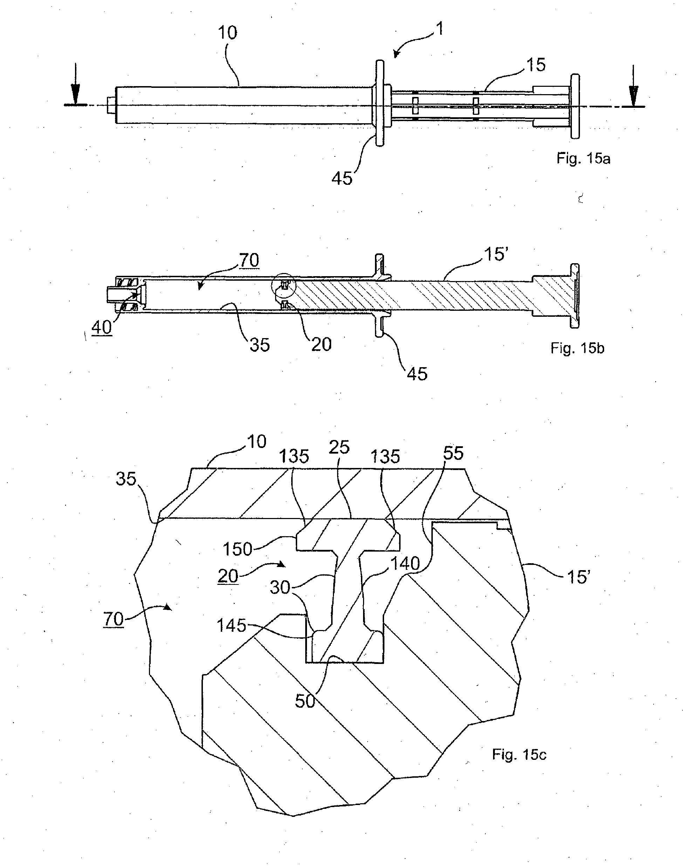

Fig. 15a, 15b, 15c zeigen jeweils ein siebtes Ausführungsbeispiel einer erfindungsgemäßen Spritze in Ansicht, Querschnitt und Ausschnittsvergrößerung in einem Zustand mit ungespanntem Federelement.

Auch das siebte Ausführungsbeispiel entspricht weitgehend dem ersten Ausführungsbeispiel. Wie schon beim ersten Ausführungsbeispiel umfasst auch die Spritze 1, die in einer Ansicht in Fig. 15a gezeigt ist, eine Hülse 10 und einen Kolben 15, wobei die Hülse an dem ihrem der Auslassöffnung gegenüberliegenden Ende einen Griff 45 aufweist, der eine Bedienung der Spritze 1 erleichtert, insbesondere beim Eindrücken und Herausziehen des Kolben 15. Ausgehend von dem der Auslassöffnung der Hülse 10 gegenüberliegenden Ende ist der Kolben 15 in der Hülse 10 aufgenommen.

Fig. 15b zeigt eine Querschnittsansicht der Spritze 1 gemäß dem siebten Ausführungsbeispiel, wie sie in Fig. 15a mit Pfeilen angedeutet ist. Die Spritze 1 umfasst die Hülse 10 mit der Innenwandung 35, die einen Hülseninnenraum 70 definiert. Die Hülse 10 ist zudem mit einer Auslassöffnung 40 versehen. Auf den Kolbenkörper 15' ist ein Reibschlussfederelement 20 aufgezogen, so dass sich der Kolbenkörper 15' durch das Reibschlussfederelement 20 hindurch erstreckt, das in Form eines Profilringes vorgesehen ist. Die Spitze des Kolbenkörpers 15' ist an die Form der Auslassöffnung angepasst, so dass es hier beim Aufnehmen von Material in die Spritze 1 zu einer möglichst geringen Luftaufnahme kommt.

Fig. 15c zeigt eine Ansichtsvergrößerung der Darstellung von Fig. 15b. Der Bereich der Vergrößerung ist in Fig. 15b durch einen Kreis angedeutet. Wie in Fig. 15c zu sehen, ist das Reibschlussfederelement 20 in Form eines Profilrings mit im Wesentlichen H-förmigem Querschnitt vorgesehen. Das Reibschlussfederelement 20 ist in einer Ausnehmung 50 des Kolbenkörpers 15' aufgenommen und somit (zusätzlich zu einer bestehenden Reibung zwischen dem Reibschlussfederelement 20 und dem Kolbenkörper 15' auch) formschlüssig gegen eine Verschiebung entlang der Längsachse der Spritze 1 gesichert. Der Kolbenkörper 15' umfasst zudem eine Kante 55, die als Anschlag dazu ausgestaltet ist, eine Verformung des Reibschlussfederelements 20 entlang der Längsachse der Spritze 1 zu begrenzen. In der Darstellung von Fig. 15c ist das Reibschlussfederelement 20 in entspanntem Zustand gezeigt und somit ist die Kante 55 von dem Reibschlussfederelement 20 beabstandet.

Das Reibschlussfederelement 20 umfasst ein an der Innenwandung 35 der Hülse anliegendes Reibschlusselement 25 und ein Federelement 30. Bei der im wesentlichen H-förmigen Ausformung des das Reibschlussfederelement bildenden Profilrings stellt der außenliegende Profilschenkel 150 das Reibschlusselement 25 dar, während das Federelement 30 von dem Profilquerschenkel 140 und dem innenliegenden Profilschenkel 145 gebildet wird. Der innenliegende Profilschenkel 145 besitzt entlang der Längsachse der Spritze 1 eine geringere Ausdehnung als der außenliegende Profilschenkel 150. Mit anderen Worten ist der innenliegenden Profilschenkel kürzer als der außenliegende, so dass sich ein "verzerrtes" H als Form ergibt. Zudem verjüngt sich der Profilquerschenkel 140 vom innenliegenden Profilschenkel 145 zum außenliegenden Profilschenkel 150 hin, also radial nach außen. Durch diese Kontur kann eine gewisse Progressivität der Federeigenschaften des Federelementes erzielt werden. Ferner ist der außenliegende Profilschenkel 150, also das Reibschlusselement 25, an den Kanten seiner Anlagefläche zur Innenfläche 35 der Hülse 10 mit Fasen 135 versehen, die ein einfacheres Einbringen des mit dem Reibschlussfederelement 20 versehenen Kolbenkörper 15' in die Hülse 10 ermöglichen. Infolge der H-Form des Profilrings weist das Reibschlusselement 30 eine in Richtung des Hülseninnenraums 70 ragende Lippe auf (vgl. auch Fig. 2d). Zudem besitzt das Reibschlusselement 25 auch eine entsprechende Lippe auf der dem Hülseninnenraum 70 abgewandten Seite. Ebenso wie bei den anderen entsprechenden Ausführungsbeispielen verhindert diese zusätzliche Dichtlippe ein Eindringen von Luft beim Zurückziehen oder Zurücktreiben des Kolbens 15, beispielsweise bei einer Füllung der Spritze.

Gemäß einer herkömmlichen Routine werden Spritzen zur Abgabe von pastösem Material zunächst montiert, wonach der Kolben dann ganz eingeschoben wird. Anschließend wird die Spritze durch die Auslassöffnung gefüllt.

Hierfür ist es von Vorteil, ein Füllrohr in die Auslassöffnung hinein zu schieben, weil so die Gefahr einer äußerlichen Verschmutzung mit Pastenbestandteilen am unwahrscheinlichsten ist.

Wird nun bei einer herkömmlichen Spritze der Kolben zusammen mit einer Dichtung durch die hineingedrückte Paste nach hinten geschoben, so kann partiell an Teilen des Umfangs Luft eingeschleppt werden. Dadurch, dass beispielsweise bei dem vorliegenden siebten Ausführungsbeispiel auch nach hinten (d.h. von der Auslassöffnung abgewandt) eine Dichtlippe vorgesehen ist, wird dieser unerwünschte Effekt verhindert.

Zudem ist das Reibschlussfederelement 20 symmetrisch gegenüber einer Vertauschung der Einbaurichtung ausgestaltet, so dass bei einer Montage von Kolbenkörper 15' und Reibschlussfederelement 20 auf eine Kontrolle der Einbaurichtung verzichtet werden kann. Dies reduziert Fehlerquellen bei der Montage und erleichtert die Montage ganz allgemein.

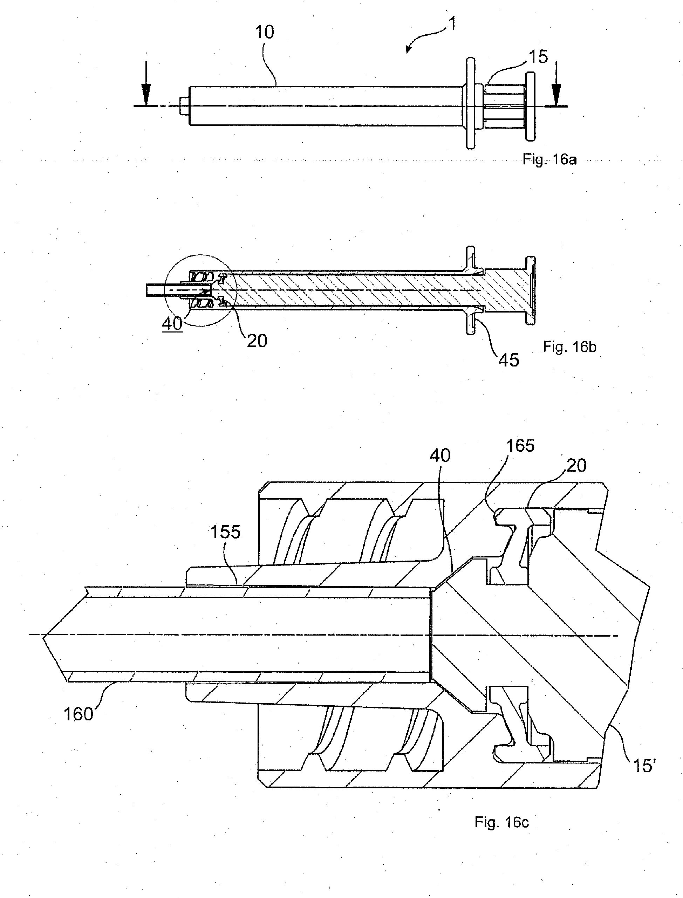

Fig. 16a, 16b, 16c zeigen jeweils das siebte Ausführungsbeispiel einer erfindungsgemäßen Spritze in Ansicht, Querschnitt und Ausschnittsvergrößerung ähnlich zu den Fig. 15a, 15b und 15c in einem Zustand mit gespanntem Federelement.

Die Darstellung in den Fig. 16a und 16b unterscheidet sich von der in den Fig. 15a und 15b darin, dass die Spritze 1 in den Figuren 16a und 16b in einem Zustand gezeigt wird, bei dem der Kolben 15 vollständig in die Hülse 10 eingebracht ist.

Fig. 16c zeigt eine vergrößerte Ausschnittsansicht der in Fig. 16b dargestellten Spritze 1, entsprechend der Andeutung durch den Kreis in Fig. 16b.

In Fig. 16c ist der Kolbenkörper 15' mit seiner sich durch das Reibschlusselement 20 erstreckenden Spitze ganz an die Auslassöffnung 40 herangeschoben. Die Hülse 10 ist hier zudem mit einer Ausnehmung 165 für den vorderen Bereich des Reibschlusselements 25 versehen, sowie so ausgestaltet, dass sich infolge des Anlegens der Spitze des Kolbenkörpers 15' an die Auslassöffnung 40 das Reibschlussfederelement 20, insbesondere mit seinem Profilquerschenkel oder Mittelschenkel an die Hülseninnenwandung im Bereich der Auslassöffnung 40 anlegt. Hiermit wird ein unerwünschtes Verbleiben von Luft im Inneren der Spritze reduziert. Mit dem Anlegen ergibt sich zudem vorliegend ein Spannen des Reibschlussfederelements.

Zudem weist die Auslassöffnung 40 einen Ansatz 155 auf, der sich in Richtung auf den Hülseninnenraum konisch verjüngt. Mit anderen Worten ist dieser Ansatz so ausgeführt, dass er sich von innen nach außen erweitert (mit einem Winkel a). Das hat den Vorteil, dass die Spritze weit auf ein Füllrohr 160 geschoben werden kann, ohne dass ungewollt Luft in den Innenraum hineinkomprimiert wird. Die Luft kann bis zum letzten Stück entweichen. Erst wenn das Abfüllrohr 160 bis zum Ende in die Öffnung 40 geschoben ist, liegt es abdichtend am gesamten Umfang an.

Eine Ausführungsform der erfindungsgemäßen Spritze weist einen hauptsächlich zylindrischen Spritzenkörper (Hülse) mit einer offenen Rückseite und einer Auslassöffnung auf. In diesem Spritzenkörper befindet sich ein translatorisch verschiebbarer Stempel (Kolben), der mit einer Dichtung (Reibschlussfederelement) gegenüber der inneren Mantelfläche (Innenwandung der Hülse) abdichtend wirkt. Die Dichtung und die Mantelfläche weisen zusammen einen hohen Reibwert oder Reibungskoeffizienten auf, d.h. ein Verschieben der Dichtung an der Mantelfläche ist nur mit einem gewissen Kraftaufwand möglich.

Die Dichtung weist einen dünnwandigen, elastisch verformbaren Bereich in Form einer Membrane (Federelement) auf. Bei Ausüben eines Druckes auf das Ende des Stößels (Kolben) wird dieser vorwärts bewegt und dabei Material bzw. Werkstoff aus der Austragöffnung oder Auslassöffnung der Spritzenkammer (Hülseninnenraum) extrudiert. Da die Dichtung (genauer das an der Innenwandung anliegende Reibschlusselement der Dichtung) aufgrund der Reibverhältnisse sich nicht ohne weiteres an der inneren Mantelfläche der Spritzenwand bewegen kann, wird zunächst der Membranbereich elastisch gestreckt, was eine Spannung des Federelements darstellt. Dies geschieht bis zu dem Punkt, an dem die Haftreibungskraft zwischen Dichtung und Innenwand des Spritzenkörpers und die Federkraft der Membrane gleich groß sind. Nun setzt sich auch die Dichtung relativ zur inneren Mantelfläche des Spritzenkörpers gleitend in Bewegung.

Das Ausmaß der verbleibenden Spannung des Federelement richtet sich nun nach der Gleitreibungskraft, die beim Gleiten des Reibschlusselements entlang der Innenwandung der Hülse auftritt.

Wird der Stößel nun entlastet, erfolgt also ein weiteres Eindrücken des Kolbens in die Hülse, unterbleibt zunächst die Bewegung zwischen äußerem Umfang der Dichtung und innerer Mantelfläche des Spritzenkörpers. Das Federelement verbleibt dabei in einem gespannten Zustand, der sich aus der Gleitreibungskraft bestimmt, die zwischen Reibschlusselement und Innenwandung der Hülse bestand. Da die das Eindringen verursachende Kraft nicht mehr auf den Kolben wirkt, wird sich anschließend die Membrane elastisch in ihre Ursprungsposition zurückziehen.

Das Federelement entspannt sich somit, wobei der Kolbenkörper entgegen der Austragrichtung zurück bewegt wird, wobei die Membran mitgenommen wird. Dadurch entsteht nicht nur eine Entlastung des in der Spritze enthaltenen Materials, sondern darüber hinaus ein Rücksaugeffekt durch die sich dabei ergebende Vergrößerung des Hülseninnenraums. Dieser Effekt ist insbesondere wünschenswert, da sich nach der Applikation in der Regel eine geringe Menge Werkstoff (beispielsweise Dentalmaterial) als kleiner Tropfen an der Kanülenspitze befindet. Dieser Tropfen verschwindet wegen dieses Effektes wieder in der Kanüle, er wird eingesaugt.

Um die elastische Verformung der Membrane auf einen exakten Wert zu begrenzen, kann der Stößel eine Kante zur Bewegungsbegrenzung aufweisen. Diese Kante kann den äußeren Dichtungsbereich (Reibschlusselement) bewegen, schon bevor die Federkraft der Membrane die Haftreibkraft von Dichtung und innerer Mantelfläche der Spritzenkörper überwindet. Auf diese Weise kann die Spannkraft des Federelements auch auf einen Wert beschränkt werden, der unterhalb der Gleitreibungskraft von Hülseninnenwandung und Reibschlusselement liegt.

Ergänzend ergibt sich ein Vorteil darin, dass mit Hilfe beispielsweise dieser Kante somit auch die Größe des Rücksaugeffektes von Fertigungsschwankungen der Dichtung (Elastizität des eingesetzten Rohstoffes, Wanddickenvariationen) unabhängig eingestellt wird.

## Patentansprüche

1. Spritze (1) zum dosierten Abgeben von Werkstoffen, insbesondere von fließfähigen und/oder pastösen dentalen Werkstoffen, mit
- einer Hülse (10) zur Aufnahme von Werkstoff und
- einem in die Hülse (10) einbringbaren Kolben (15) mit:
- einem Kolbenkörper (15'),
- einem Reibschlusselement (25), das an einer Innenwandung (35) der Hülse (10) reibschlüssig anliegt, und
- einem das Reibschlusselement (25) und den Kolbenkörper (15') koppelnden Federelement (30),
wobei die Hülse (10) und der in die Hülse (10) eingebrachte Kolben (15) einen Hülseninnenraum (70) zur Aufnahme des Werkstoffs begrenzen,
wobei das Federelement (30) bei einem Einbringen des Kolbens (15) und/oder des Kolbenkörpers (15') in die Hülse (10) durch ein relatives Zurückbleiben des Reibschlusselementes (25) gegenüber dem Kolbenkörper (15') spannbar ist, wobei durch eine Entspannung des Federelements (30) ein zumindest teilweises Austreiben des Kolbenkörpers (15') aus der Hülse (10) bei unbewegtem Reibschlusselement (25) zur Vergrößerung des Hülseninnenraums (70) bewirkbar ist, wobei das Reibschlusselement (25) und die Innenwandung (35) für eine gegenseitige Reibungskraft zur Bewirkung des relativen Zurückbleibens des Reibschlusselements (25) ausgestaltet sind, **gekennzeichnet durch** ein Begrenzungsmittel (55, 60, 65) zur Begrenzung einer Relativverschiebung zwischen Reibschlusselement (25) und Kolbenkörper (15') auf eine vorbestimmte Entfernung von einer relativen Ausgangsposition.

2. Spritze (1) nach Anspruch 1,
wobei das Federelement (30) und das Reibschlusselement (25) gemeinsam einstückig als Reibschlussfederelement (20) ausgebildet sind, wobei das Reibschlussfederelement (20) einen Profilring mit im Wesentlichen H-, T- oder L-förmigem Querschnitt aufweist.

3. Spritze (1) nach Anspruch 2.
wobei das Reibschlussfederelement (20) einen Profilring mit im Wesentlichen H-förmigen Querschnitt aufweist, wobei der außenliegende Profilschenkel das Reibschlusselement (25) und der innenliegende Profilschenkel und der Profilquerschenkel das Federelement (30) bilden und wobei das Federelement (30) an einer Außenfläche des Kolbenkörpers (15') anliegt.

4. Spritze (1) nach Anspruch 3,
wobei das Federelement (30) in einer Ausnehmung (50) des Kolbenkörpers (15') anliegt.

5. Spritze (1) nach einem der vorstehenden Ansprüche,
wobei das Federelement (30) bei in die Hülse (10) eingebrachtem Kolben (15) durch das Reibschlusselement (25) von der Innenwandung (35) der Hülse (10) beabstandet ist.

6. Spritze (1) nach einem der vorstehenden Ansprüche,
wobei das Federelement (30) eine Öffnung, insbesondere eine mittige Öffnung, aufweist, durch die sich ein erster Teil (85) des Kolbenkörpers erstreckt (15'), wobei der erste Teil (85) des Kolbenkörpers (15') und ein zweiter Teil (90) des Kolbenkörpers (15') zum Einspannen des Federelements (30) im Kolbenkörper (15') miteinander verbunden sind.

7. Spritze (1) nach Anspruch 1,
mit einem ersten Begrenzungselement (55) zur Begrenzung der Relativverschiebung beim Einbringen des Kolbens (15) und/oder des Kolbenkörpers (15') in dje Hülse (10), insbesondere eine Stufe oder Kante (55), an die das Reibschlusselement (25) und/oder das Federelement (30) bei gespanntem Federelement (30) anlegbar sind.

8. Spritze (1) nach einem der Ansprüche 1 oder 7,
mit einem zweiten Begrenzungselement (60, 65) zur Begrenzung der Relativverschiebung bei einem zumindest teilweisen Entfernen des Kolbens (15) und/oder des Kolbenkörpers (15') aus der Hülse (10), wobei der Kolbenkörper (15') insbesondere mit einem ersten Vorsprung (60) versehen ist, der einen zweiten Vorsprung (65) des Reibschlusselements (25) und/oder des Federelements (30) hintergreift, so dass der erste Vorsprung (60) bei dem Entfernen des Kolbens (15) und/oder des Kolbenkörpers (15') an den zweiten Vorsprung (65) anlegbar ist.

9. Spritze (1) nach Anspruch 3 oder 4.
wobei der innenliegende Profilschenkel des Reibschlussfederelements (20) eine geringere Ausdehnung in Längsrichtung der Spritze (1) aufweist als der außenliegende Prafilschenkel (25).

10. Spritze (1) nach Anspruch 3, 4 oder 9,
wobei der Profilquerschenkel des Reibschlussfederelements (20) eine Dicke in Längsrichtung der Spritze (1) aufweist, die in radialer Richtung nach außen abnimmt.

11. Spritze (1) nach einem der vorstehenden Ansprüche.
wobei die Hülse (10) eine Auslassöffnung (40) mit einem Ansatz zur Aufnahme eines Füllrohres aufweist, wobei der Ansatz sich von außen in Richtung auf den Hülseninnenraum (70) verjüngt.

12. Kolben (15) für eine Spritze (1) zum dosierten Abgeben von Werkstoffen, insbesondere von flleßfähigen und/oder pastösen dentalen Werkstoffen, bevorzugt für eine Spritze (1) nach einem der Ansprüche 1 bis 12.
wobei der Kolben (15) in die Spritze (1) einbringbar ist und aufweist:
- einen Kolbenkörper (15'),
- ein Relbschlusselement (25), das an einer Innenwandung (35) einer Hülse (10) der Spritze (1) reibschlüssig anlegbar ist, und
- ein das Reibschlusselement (25) und den Kolbenkörper (15') koppeindes Federelement (30),
wobei der Kolben (15) ausgestaltet ist, bei in die Hülse (10) eingebrachtem Kolben (15) mit der Hülse (10) einen Hülseninnenraum (70) zur Aufnahme des Werkstoffs zu begrenzen.
wobei das Federelement (30) bei einem Einbringen des Kolbens (15) und/oder des Kolbenkörpers (15') in die Hülse (10) durch ein relatives Zurückbleiben des Reibschlusselementes (25) gegenüber dem Kolbenkörper (15') spannbar ist, wobei durch eine Entspannung des Federelements (30) ein zumindest tellweises Austreiben des Kolbenkörpers (15') aus der Hülse (10) bei unbewegtem Reibschlusselement (25) zur Vergrößerung des Hülsenninenraums (70) bewirkbar ist, wobei das Reibschlusselement (25) für eine Reibungskraft gegenüber der Innenwandung (35) zur Bewirkung des relativen Zurückbleibens des Reibschlusselements (25) ausgestaltet ist, **gekennzeichnet durch** ein Begrenzungsmittel (55, 60, 65) zur Begrenzung einer Relativverschiebung zwischen Reibschluswelement (25) und Kolbenkörper (15') auf eine vorbestimmte Entfernung von einer relativen Ausgangsposition.

13. Reibschlussfederelement (20) umfassend ein Reibschluss- und ein Federelement (25, 30) für eine Spritze (1) nach einem der Ansprüche 1 bis 11.

14. Verfahren zum dosierten Abgeben von Werkstoffen, insbesondere von fließfähigen und/oder pastösen dentalen Werkstoffen, mit einer Spritze (1) mit:
- einer Hülse (10) zur Aufnahme vom Werkstoff und
- einem in die Hülse (10) einbringbaren Kolben (15) mit:
- einem Kolbenkörper (15'),
- einem Reibschlusselement (25), das an einer Innenwandung (35) der Hülse (10) reibschlüssig anliegt, und
- einem das Reibschlusselement (25) und den Kolbenkörper (15') koppelndes Federelement (30),
wobei die Hülse (10) und der in die Hülse (10) eingebrachte Kolben (15) einen Hülseninnenraum (70) zur Aufnahme des Werkstoffs begrenzen,
mit den Schritten:
- Bereitstellen (100-110) einer zumindest teilweise mit Werkstoff gefüllten Spritze (1),
- Beaufschlagen (115) des Kolbens (15) mit einer Kraft zum Einbringen des Kolbens (15) und/oder des Kolbenkörpers (15') in die Hülse (10), um dosiert Werkstoff aus der Spritze (1) abzugeben,
- Spannen (120) des Federelementes (30) durch ein relatives Zurückbleiben des Reibschlusselements (25) gegenüber dem Kolbenkörper (15'),
- Beenden (125) der Kraftbeaufschlegung des Kolbens (15),
- zumindest teilweises Austreiben (130) des Kolbenkörpers (15') aus der Hülse (10) bei unbewegtem Reibschlusselement (25) durch Entspannen des Federelements (30), wobei durch das Austreiben (130) eine Vergrößerung des Hülseninnenraums (70) bewirkt wird, um ein ungewolltes weiteres Abgeben von Werkstoff zumindest teilweise zu unterbinden,
wobei das relative Zurückbleiben des Reibschlusselement (25) durch eine gegenseitige Reibungskraft zwischen dem Reibschlusselement (25) und der Innenwandung (35) bewirkt wird, **gekennzeichnet dadurch, dass** eine Relatiwerschiebung zwischen Rückschlusselement (25) und Kolbenkörper (15') durch ein Begrenzungsmittel (55, 60, 65) auf eine vorbestimmte Entfernung von einer relatifven Ausgangsposition begrenzt wird.

## Claims

1. A syringe (1) for the metered dispensing of materials, in particular flowable and/or pasty, dental materials, having:
- a casing (10) for receiving material and
- a plunger (15), insertable into the casing (10), having:
- a plunger body (15'),
- a frictional connection element (25), contacting an inner wall (35) of the casing (10) in a friction-locked manner, and
- a spring element (30) coupling the frictional connection element (25) and the plunger body (15'),
wherein the casing (10) and the plunger (15) inserted into the casing (10) bound a casing interior (70) for receiving the material,
wherein upon insertion of the plunger (15) and/or the plunger body (15') into the casing (10), the spring element (30) is tensionable by a relative lagging behind of the frictional connection element (25) with respect to the plunger body (15'), wherein by a relaxation of the spring element (30), an at least partial driving out of the plunger body (15') from the casing (10) accompanied by non-movement of the frictional connection element (25) can be effected to enlarge the casing interior (70), wherein the frictional connection element (25) and the inner wall (35) are fashioned for a mutual frictional force for bringing about the relative lagging behind of the frictional connection element (25), **characterised by** a limiting means (55, 60, 65) for limiting a relative displacement between frictional connection element (25) and plunger body (15') to a predetermined distance from a relative initial position.

2. A syringe (1) according to claim 1, wherein the spring element (30) and the frictional connection element (25) are jointly formed in one piece as a frictional connection spring element (20), wherein the frictional connection spring element (20) has a shaped ring of substantially H-, T- or L-shaped cross-section.

3. A syringe (1) according to claim 2, wherein the frictional connection spring element (20) has a shaped ring of substantially H-shaped cross-section, wherein the outer shaped leg forms the frictional connection element (25) and the inner shaped leg and the shaped transverse leg form the spring element (30) and wherein the spring element (30) contacts an outer surface of the plunger body (15').

4. A syringe (1) according to claim 3, wherein the spring element (30) contacts in a cutout (50) of the plunger body (15').

5. A syringe (1) according to any one of the preceding claims, wherein with the plunger (15) inserted into the casing (10), the spring element (30) is spaced apart from the inner wall (35) of the casing (10) by the frictional connection element (25).

6. A syringe (1) according to any one of the preceding claims, wherein the spring element (30) has an opening, in particular a central opening, through which a first part (85) of the plunger body extends (15'), wherein the first part (85) of the plunger body (15') and a second part (90) of the plunger body (15') are connected to one another for clamping the spring element (30) in the plunger body (15').

7. A syringe (1) according to claim 1, having a first limiting element (55) for limiting the relative displacement upon insertion of the plunger (15) and/or the plunger body (15') into the casing (10), in particular a step or edge (55) against which the frictional connection element (25) and/or the spring element (30) can make contact when the spring element (30) is tensioned.

8. A syringe (1) according to one of claims 1 or 7, having a second limiting element (60, 65) for limiting the relative displacement in the case of an at least partial removal of the plunger (15) and/or the plunger body (15') from the casing (10), wherein the plunger body (15') is in particular provided with a first projection (60) engaging behind a second projection (65) of the frictional connection element (25) and/or the spring element (30), so that the first projection (60) can make contact against the second projection (65) upon removal of the plunger (15) and/or the plunger body (15').

9. A syringe (1) according to claim 3 or 4, wherein the inner shaped leg of the frictional connection spring element (20) has a shorter extension in the longitudinal direction of the syringe (1) than the outer shaped leg (25).

10. A syringe (1) according to claim 3, 4 or 9, wherein in the longitudinal direction of the syringe (1), the shaped transverse leg of the frictional connection spring element (20) has a thickness which decreases outwards in a radial direction.

11. A syringe (1) according to any one of the preceding claims, wherein the casing (10) has an outlet opening (40) with an attachment for receiving a filling pipe, wherein the attachment tapers from the exterior in the direction of the casing interior (70).

12. A plunger (15) for a syringe (1) for the metered dispensing of materials, in particular flowable and/or pasty, dental materials, preferably for a syringe (1) according to any one of claims 1 to 12,
wherein the plunger (15) can be inserted into the syringe (1) and has:
- a plunger body (15'),
- a frictional connection element (25) which can make contact against an inner wall (35) of a casing (10) of the syringe (1) in a friction-locked manner, and
- a spring element (30) coupling the frictional connection element (25) and the plunger body (15'),
wherein the plunger (15) is constructed to bound, together with the casing (10) and when the plunger (15) has been inserted into the casing (10), a casing interior (70) for receiving the material,
wherein the spring element (30) is, upon insertion of the plunger (15) and/or the plunger body (15') into the casing (10), tensionable by a relative lagging behind of the frictional connection element (25) with respect to the plunger body (15'), wherein by a relaxation of the spring element (30), an at least partial driving out of the plunger body (15') from the casing (10) accompanied by non-movement of the frictional connection element (25) can be effected to enlarge the casing interior (70), wherein the frictional connection element (25) is fashioned for a frictional force with respect to the inner wall (35) to effect the relative lagging behind of the frictional connection element (25), **characterised by** a limiting means (55, 60, 65) to limit a relative displacement between frictional connection element (25) and plunger body (15') to a predetermined distance from a relative initial position.

13. A frictional connection spring element (20) comprising a frictional connection element and a spring element (25, 30) for a syringe (1) according to any one of claims 1 to 11.

14. A method for the metered dispensing of materials, in particular flowable and/or pasty, dental materials, with a syringe (1) having:
- a casing (10) for receiving the material and
- a plunger (15), insertable into the casing (10), having:
- a plunger body (15'),
- a frictional connection element (25), contacting an inner wall (35) of the casing (10) in a friction-locked manner, and
- a spring element (30) coupling the frictional connection element (25) and the plunger body (15'),
wherein the casing (10) and the plunger (15) inserted in the casing (10) bound a casing interior (70) for receiving the material,
having the steps:
- provision (100-110) of a syringe (1) at least partially filled with material,
- action (115) upon the plunger (15) with a force to introduce the plunger (15) and/or the plunger body (15') into the casing (10), in order to dispense material from the syringe (1) in a metered manner,
- tensioning (120) of the spring element (30) through a relative lagging behind of the frictional connection element (25) with respect to the plunger body (15'),
- ending (125) of the action of force upon the plunger (15),
- at least partial driving out (130) of the plunger body (15') from the casing (10), accompanied by non-movement of the frictional connection element (25), by relaxation of the spring element (30), wherein through the driving out (130) there is effected an enlargement of the casing interior (70) in order to at least partially prevent an undesired further dispensing of material,
wherein the relative lagging behind of the frictional connection element (25) is effected by a mutual frictional force between the frictional connection element (25) and the inner wall (35), **characterised in that** a relative displacement between frictional connection element (25) and plunger body (15') is limited by a limiting means (55, 60, 65) to a predetermined distance from a relative initial position.

## Revendications

1. Seringue (1) pour administrer de manière dosée des substances actives, en particulier des substances actives dentaires coulantes et/ou pâteuses, comprenant :
- une douille (10) pour recevoir une substance active ; et
- un piston (15) pouvant être inséré dans la douille (10), comprenant :
- un corps de piston (15') ;
- un élément de liaison par friction (25) qui repose par friction contre une paroi intérieure (35) de la douille (10) ; et
- un élément formant ressort (30) couplant l'élément de liaison par friction (25) et le corps de piston (15'),
dans laquelle la douille (10) et le piston (15) inséré dans la douille (10) délimitent un espace intérieur de douille (70) pour recevoir la substance active,
dans laquelle l'élément formant ressort (30) peut être tendu, lorsque le piston (15) et/ou le corps de piston (15') sont insérés dans la douille (10), par un retrait relatif de l'élément de liaison par friction (25) par rapport au corps de piston (15'), la détente de l'élément formant ressort (30) permettant d'entraîner l'expulsion au moins en partie du corps de piston (15') hors de la douille (10), lorsque l'élément de liaison par friction (25) est immobile, afin d'agrandir l'espace intérieur de douille (70),
dans laquelle l'élément de liaison par friction (25) et la paroi intérieure (35) sont configurés pour une force de friction mutuelle pour entraîner le retrait relatif de l'élément de liaison par friction (25), **caractérisée par** un moyen de limitation (55, 60, 65) pour limiter un déplacement relatif par coulissement entre l'élément de liaison par friction (25) et le corps de piston (15') à une distance prédéterminée de la position relative de départ.

2. Seringue (1) selon la revendication 1,
dans laquelle l'élément formant ressort (30) et l'élément de liaison par friction (25) sont réalisés en commun d'un seul tenant sous la forme d'un élément formant ressort de liaison par friction (20), l'élément formant ressort de liaison par friction (20) présentant une bague profilée de section transversale présentant essentiellement une forme de H,, de T ou de L.

3. Seringue (1) selon la revendication 2,
dans laquelle l'élément formant ressort de liaison par friction (20) présente une bague profilée de section transversale présentant essentiellement une forme de H, la branche profilée située à l'extérieur formant l'élément de liaison par friction (25) et que la branche profilée située à l'intérieur et la branche transversale profilée forment l'élément formant ressort (30) et l'élément formant ressort (30) reposant contre une surface extérieure du corps de piston (15').

4. Seringue (1) selon la revendication 3,
dans laquelle l'élément formant ressort (30) repose dans un évidement (50) du corps de piston (15').

5. Seringue (1) selon l'une quelconque des revendications précédentes,
dans laquelle l'élément formant ressort (30) est espacé de la paroi intérieure (35) de la douille (10) par l'élément de liaison par friction (25) lorsque le piston (15) est inséré dans la douille (10).

6. Seringue (1) selon l'une quelconque des revendications précédentes,
dans laquelle l'élément formant ressort (30) présente une ouverture, en particulier une ouverture centrale, à travers laquelle une première partie (85) du corps de piston (15') s'étend, la première partie (85) du corps de piston (15') et une deuxième partie (90) du corps de piston (15') étant reliées l'une à l'autre pour tendre l'élément formant ressort (30) dans le corps de piston (15').

7. Seringue (1) selon la revendication 1,
comprenant un premier élément de limitation (55) pour limiter le déplacement relatif par coulissement lors de l'introduction du piston (15) et/ou du corps de piston (15') dans la douille (10), en particulier comprenant un palier ou une arête (55), contre lequel/ laquelle l'élément de liaison par friction (25) et/ou l'élément formant ressort (30) peuvent être placés lorsque l'élément formant ressort (30) est tendu.

8. Seringue (1) selon l'une quelconque des revendications 1 ou 7,
comprenant un deuxième élément de limitation (60, 65) pour limiter le déplacement relatif par coulissement lorsque le piston (15) et/ou le corps de piston (15') sont retirés au moins en partie de la douille (10), le corps de piston (15') étant pourvu en particulier d'une première partie faisant saillie (60), laquelle vient en prise par l'arrière avec une deuxième partie faisant saillie (65) de l'élément de liaison par friction (25) et/ou de l'élément formant ressort (30) de sorte que la première partie faisant saillie (60) peut être placée contre la deuxième partie faisant saillie (65) lors du retrait du piston (15) et/ou du corps de piston (15').

9. Seringue (1) selon la revendication 3 ou 4,
dans laquelle la branche profilée située à l'intérieur de l'élément formant ressort de liaison par friction (20) présente une extension moindre dans la direction longitudinale de la seringue (1) que la branche profilée située à l'extérieur (25).

10. Seringue (1) selon la revendication 3, 4 ou 9, dans laquelle la branche transversale profilée de l'élément formant ressort de liaison par friction (20) présente une épaisseur dans la direction longitudinale de la seringue (1), laquelle diminue dans la direction radiale vers l'extérieur.

11. Seringue (1) selon l'une quelconque des revendications précédentes,
dans laquelle la douille (10) présente une ouverture d'évacuation (40) présentant un épaulement pour recevoir un tube de remplissage, l'épaulement se rétrécissant de l'extérieur dans la direction de l'espace intérieur de douille (70).

12. Piston (15) pour une seringue (1) pour administrer de manière dosée des substances actives, en particulier des substances actives dentaires coulantes et/ou pâteuses, de préférence pour une seringue (1) selon l'une quelconque des revendications 1 à 12,
le piston (15) pouvant être inséré dans la seringue (1) et présentant
- un corps de piston (15'),
- un élément de liaison par friction (25), qui peut être placé par friction contre une paroi intérieure (35) d'une douille (10) de la seringue (1), et
- un élément formant ressort (30) couplant l'élément de liaison par friction (25) et le corps de piston (15'),
le piston (15) étant configuré pour limiter, avec la douille (10), un espace intérieur de douille (70) pour recevoir la substance active, lorsque le piston (15) est inséré dans la douille (10),
dans lequel l'élément formant ressort (30) peut être tendu, lorsque le piston (15) et/ou le corps de piston (15') sont insérés dans la douille (10), par un retrait relatif de l'élément de liaison par friction (25) par rapport au corps de piston (15'), la détente de l'élément formant ressort (30) permettant d'entraîner au moins en partie l'expulsion du corps de piston (15') hors de la douille (10) lorsque l'élément de liaison par friction (25) est immobile afin d'agrandir l'espace intérieur de douille (70),
dans lequel l'élément de liaison par friction (25) est configuré pour une force de friction par rapport à la paroi intérieure (35) afin d'entraîner le retrait relatif de l'élément de liaison par friction (25), **caractérisé par** un moyen de limitation (55, 60 ,65) pour limiter un déplacement relatif par coulissement entre l'élément de liaison par friction (25) et le corps de piston (15') à une distance prédéterminée d'une position relative de départ.

13. Elément formant ressort de liaison par friction (20), comprenant un élément de liaison par friction et un élément formant ressort (25, 30) pour une seringue (1) selon l'une quelconque des revendications 1 à 11.

14. Procédé pour administrer de manière dosée des substances actives, en particulier des substances actives dentaires coulantes et/ou pâteuses, à l'aide d'une seringue (1) comprenant :
- une douille (10) pour recevoir une substance active ; et
- un piston (15) pouvant être inséré dans la douille (10), comprenant :
- un corps de piston (15') ;
- un élément de liaison par friction (25), qui repose par friction contre une paroi intérieure (35) de la douille (10) ; et
- un élément formant ressort (30) couplant l'élément de liaison par friction (25) et le corps de piston (15'),
dans lequel la douille (10) et le piston (15) inséré dans la douille (10) délimitent un espace intérieur de douille (70) pour recevoir la substance active,
ledit procédé comprenant les étapes suivantes consistant à :
- fournir (100 - 110) une seringue (1) remplie au moins en partie d'une substance active ;
- soumettre (115) le piston (15) à l'action d'une force pour introduire le piston (15) et/ou le corps de piston (15') dans la douille (10) afin d'administrer de manière dosée une substance active en provenance de la seringue (1) ;
- tendre (120) l'élément formant ressort (30) par un retrait relatif de l'élément de liaison par friction (25) par rapport au corps de piston (15') ;
- mettre fin (125) à l'application de la force à laquelle est soumis le piston (15) ;
- expulser (130) au moins en partie le corps de piston (15') hors de la douille (10) lorsque l'élément de liaison par friction (25) est immobile en détendant l'élément formant ressort (30), l'expulsion (130) entraînant un agrandissement de l'espace intérieur de douille (70) afin d'empêcher au moins en partie toute administration supplémentaire non souhaitée d'une substance active,
dans lequel le retrait relatif de l'élément de liaison par friction (25) est entraîné par une force de friction mutuelle entre l'élément de liaison par friction (25) et la paroi intérieure (35), **caractérisé en ce qu'**un déplacement relatif par coulissement entre l'élément de liaison par friction (25) et le corps de piston (15') est limité par un moyen de limitation (55, 60, 65) à une distance prédéterminée d'une position relative de départ.
